# EUROPEAN PATENT SPECIFICATION

(11) **EP 2 730 585 B1**
(45) Date of publication and mention of the grant of the patent: **11.04.2018**
(21) Application number: 12793628.4
(22) Date of filing: 28.02.2012
(51) Int. Cl.: A61K 35/54, A61K 35/36, A61K 35/30, A61K 38/02, A61Q 19/08, A61K 8/64, A61K 8/98

(54) **PROTEIN-POLYPEPTIDE COMPLEX OBTAINED FROM TISSUES OF EMBRYOS OF HOOFED ANIMALS , USE ON SKIN TISSUE, METHOD FOR PRODUCING SAID COMPLEX AND PHARMACEUTICAL COMPOSITION**
PROTEIN-POLYPEPTID-KOMPLEX AUS GEWEBE VON HUFTIEREMBRYONEN, VERWENDUNG AUF DIE HAUT, VERFAHREN ZUR HERSTELLUNG DIESES KOMPLEXES UND PHARMAZEUTISCHE ZUSAMMENSETZUNG DARAUS
COMPLEXE DE PROTÉINE-PEPTIDE PROVENANT DE TISSUS D'EMBRYONS D'ONGULÉS, UTILISATION SUR LA PEAU, PROCÉDÉ DE PRODUCTION ET COMPOSITION PHARMACEUTIQUE LE COMPRENANT

(30) Priority: 30.05.2011 RU 2011121684
(43) Date of publication of application: 14.05.2014
(73) Proprietor: Aktsionernoe Obschestvo "Pharm-Sintez", Moskow, 111024 (RU)
(72) Inventor: NAZARENKO, Anna Borisovna, Moscow 121614 (RU); SOKOLOV, Mikhail Anatolevich, Moscow 107150 (RU)
(74) Representative: Held, Stephan
(86) International application number: PCT/RU2012/000141
(87) International publication number: WO 2012/166006

(56) References cited:
- WO-A1-2009/088314
- WO-A1-2010/007620
- EA-A- 200 000 634
- RU-C1- 2 142 785
- US-A1- 2006 058 238
- SPRAVOCHNIK VIDAL.: 'Lekarstvennye preparaty v Rossii.' SPRAVOCHNIK. IZDANIE XVI. M. ASTRAFARMSERVIS 2010, pages 506 - 507

## Description

### Field

The invention relates to biologically active substance prepared from nerve and skin tissues of hoofed farm embryos, intended as a drug substance in medical and beauty products stimulating physiological and reparative regeneration of skin, appearing as decrease of aging features prominence with a rejuvenescent effect and protection from environmental hazards, and the method for production of the same.

Authorized abbreviations:
PPC - protein/polypeptide complex;
PPNC - protein/polypeptide/nucleotide complex;
I - ionic strength of solution;
Pharmcomposition - pharmaceutical composition.

At present, on the background of increased impact of numerous external and internal factors, including man-made, environmental, and atmospheric disasters, increased use of synthetic organic chemicals, emotional and physical stress, endocrine disregulation, immune shift, etc., on skin barrier function with a steady increase of skin diseases of autoimmune, vascular, toxic, and traumatic origin, and iatrogenic complications related to the usage of "anti-aging" products, cosmetics, and care, there are very few natural active compounds supporting and gently stimulating physiological regeneration of skin, not only protecting it from harmful impacts but renewing it.

The purpose of this invention was to produce a biologically active substance which has an organo-specific effect on skin tissue, stimulating its physiological regeneration, regulating cell metabolic processes and having a rejuvenescent effect, and a pharmcomposition based on this substance, in the form of a protein/polypeptide/nucleotide (PPNC) complex.

The biologically active substance is a protein/polypeptide complex (PPC) with a molecular weight of its constituent protein/polypeptide components ranging from 0.5 to 200 kDa and the minimum content of medium molecular fraction within the range of 20 to 160 kDa not less than 70%, and the pharmcomposition is a PPC with a pharmaceutically acceptable buffer and addition of detergents, solubilizers, preservatives, and equal nucleotide component concentration presented by a sodium salt of desoxyribonucleic acid with a molecular weight of its constituent polynucleotides ranging from 12 to 660 kDa (18-1000 base pairs), with the minimum content of medium molecular fraction within the range of 20 to 500 kDa not less than 80%.

The PPC was obtained by ion-exchange chromatography of supernatant fraction filtrate of centrifugated homogenate of nerve and skin tissues of hoofed farm embryos of gestation stage from the middle of the first third to the middle of the last third of pregnancy, in the presence of buffer, detergents, and solubilizers.

The nucleotide fraction for the PPNC pharmcomposition was obtained by a known method of alkaline hydrolysis of tissue homogenate centrifuge effluent remaining after production of the protein fraction with further purification and double precipitation first with isopropanol then with ethanol, with centrifugation and repeated dilution for obtaining the target fraction. The fractions are associated in required concentration ratios, making the PPNC pharmcomposition, adding a pharmaceutically acceptable buffer, detergents, solubilizers, and preservatives.

### Prior Art

A number of preparations are known of protein-peptide nature, made from of raw materials of animal origin, having regenerative/reparative activity and used for treatment of skin diseases in medicine and beauty industry.

A product is known, normalizing skin tissue metabolism [1], including, apart from the fraction of low-molecular, associated with RNA proteins from nuclei and cytoplasm of any fetal tissue cells (as an example, pig brain, liver, thymus and spleen tissues were taken in different ratios), peptide fraction from pig fetal organs homogenate, produced by acid extraction of 0.15M HCl with heating on boiling water bath during 30 min and further neutralization with alkali to pH 7.0 and sediment centrifugation resulting in peptide concentration reaching 8 mg/ml.

With this method, the end product contains modified, denatured products of acid and thermal protein and polypeptide hydrolysis with modified quarternary and tertiary structure, and many active molecules decay products, which reduces its specificity and focus and defines low biological activity.

A method is known for producing a biologically active preparation from bovine blood and a pharmaceutical composition based on this preparation, having radioprotective, antihypoxic, immunomodulating, vulnerary and antiherpetic activity [2], this method allows for obtaining a deproteinized extract with sequential ultrafiltration with component weights ranging from 10 to 100 kDa. This method allows for obtaining a deproteinized extract of undetermined composition, containing low-molecular peptides, amino acids, nucleic acids derivatives, and other components. Absence of proteins as a class, represented in tissues by enzymes, growth and differentiation factors, signaling molecules, defines its low biological activity and reduces its specificity.

A method is known for producing biologically active lipophilic and hydrophilic fractions of pig placenta [3], [4]. The method allows for producing a mixture of peptides, amino acids, oxyacids by chloroform-ethanol and water-ethanol extraction with saline fractions separation, and usage of freon solvents to produce a lipophilic fraction.

This method does not allow for producing skin tissue-specific proteins and polypeptides, making a systemic effect, and the presence of growth factors without differentiation factors and signaling tissue molecules dramatically increases the risk of neoplastic processes, especially in absence of polynucleotides making a pronounced immunomodulating effect. The resulting fractions have no clear physical and chemical characteristics, and the chloroform-ethanol and water-ethanol extraction method itself provides for production of only low-molecular polypeptides and proteins with modified quarternary and tertiary structure, which inevitably leads to reduction of its specific biological activity.

An immune response modifier is known named Derinat, produced by ZAO Pharmaceutical Company "Technomedservis" (Russia) [5], making an impact on cell-mediated 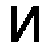 antibody-mediated (humoral) immunity, stimulating reparative processes and hematopoiesis (normalizing the count of granulocytes, lymphocyte, thrombocytes), making an anti-inflammatory and antineoplastic effect, normalizing tissue condition in cases of dystrophic changes of vascular origin, making a slight anticoagulative effect. Derinat polynucleotide composition in the form of sodium salt of desoxyribonucleic acid (Sodium deoxyribonucleate) designates the product's main systemic effect as an immunomodulator impacting cell-mediated 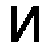 antibody-mediated immunity. The preparation stimulates reparative processes and hematopoiesis (normalizing the count of granulocytes, lymphocyte, thrombocytes), making an anti-inflammatory and antineoplastic effect, normalizing tissue condition in cases of dystrophic changes of vascular origin, making a slight anticoagulative effect. In cases of leukopaenia resulting from polychemotherapy or radiological therapy, leukopoiesis stimulation is observed even after a single injection. Usage of the preparation within the first 24 hours upon radiation exposure speeds up the start and the rate of stem cells, myeloid, lymphoid, and thrombocytic hematopoietic lineages repair. The preparation activates antiviral, antimycotic and antimicrobial immunity, increases phagocyte activity to Chlamydiaceae, Staphylococcus aureus, Escherichia coli, Helicobacter pylori. Derinat significantly reduces cell sensitivity to the damaging effect of cytotoxics and radiotherapy, which shows itself in reduction of cardio- and myelotoxicity in oncology patients and the therapeutic effect stability in retreatment. The preparation makes a non-specific reparative and regenerative effect. The disadvantage of the known product is its low specificity and, consequently, low regenerative effect, including the reparative potential for skin tissue.

There is a medicinal product of bionormalizing action and the method for producing the same [6], the active ingredient being placenta extract, produced by oxidation-hydrolitic modification with chlorous acid solution with tissue/chlorous acid ratio being 1:(0.1-1.5) and modifying agents (organic compounds of nickel or cobalt), providing for the process selectivity. The medicinal product contains polypeptides in the amount of 3.5-7.0 % wt, amino acids in the amount of 50-60 % wt, aminosaccharides in the amount of 4-5 % wt and hexuronic acids in the amount of 8-9 % wt, therewith the said products are in oxidized water-soluble for and the rest are polysaccharides and inorganic acids.

With this method, the end product contains modified, denatured products of acid protein and polypeptide hydrolysis with modified quarternary and tertiary structure, and many active molecules decay products, which reduces its specificity and focus, defines low biological activity and increases its immunological potency.

A method is known for producing a biologically active preparation [7], allowing to obtain a midgestation cow placenta extract by homogenizing in amniotic fluid with further separation of the target fraction by centrifugation.

The resulting product does not contain skin tissue-specific biologically active molecules. The extract produced by the above method includes a wide set of biological agents, including hormones, different-calibre proteins, fats, lipopolysaccharides, glycoproteids, etc., which can account for the extract's high immunological and allergenic potency.

A method is known for producing a low-molecular fraction from pig placenta tissues [8], allowing to obtain fractions with molecular weights ranging from 300 to 350 kDa by lyophilization at temperatures ranging from (-10°) to (-20°C) and residual chamber pressure not exceeding 0.1 mm Mercury for 16-20 hours.

The resulting fraction contains many placenta tissue constituents, which is not specific for skin tissue, and absence of proteins represented in tissues by enzymes, growth and differentiation factors, and signaling molecules, defines its low biological activity.

There is a known biologically active product, a method for producing the same, a preparation containing the said product, and a method for application of the said preparation [9], containing thermostable, low-molecular modified components of cell membranes with antigenic structure modified during embryogenesis and/or proliferation, and/or cell differentiation, and/or pathology preceding or accompanying tissue regeneration and/or reparation by hydrolysis of centrifugated sediment of modified animal tissue homogenization (fetal, taken before the middle of gestation) with further ultrafiltration and sterilization of the resulting supernatant; for preparation, a composition from the preparation is used, containing modified cell membrane components and a filler.

Cell membrane components are strong immunogens, which biological activity of this product that stimulates immunity non-specifically. Absence of biologically active molecules represented in tissues by enzymes, growth and differentiation factors, and signaling molecules, defines its low specific activity.

There is a known product for biological skin rejuvenation using a substance based on stabilized emulsion of perfluorocarbons, allowing for managing metabolic processes in skin tissues in direction opposite to the natural aging processes [10].

This product allows to slightly intensify skin tissue building agents synthesis - collagen, keratin, elastin. However, in order to achieve an undoubted rejuvenescent effect, a skin tissue-specific epigenetic impact is necessary, either on telomerase activation processes with Hayflick interval increase or on expression of factors involved in cell reprogramming for achieving a certain pluripotency degree, which, uncontrolled by differentiation factors, may induce neoplastic processes and lead to oncological diseases.

There is a known method for biological skin rejuvenation [11], involving intradermal and subcutaneous administration of dispersed bio-compatible material "Alloplant" [12] having an ability to attract the macrophage range cells with simulation of their maturation, skin cover acid-alkali rebalancing through sweat and oil glands functioning normalization, with metabolic processes change to prevalence of the processes that are characteristic of a physiologically younger tissue. This results in the ability to correct fibrous and degenerative-dystrophic changes in tissues (the ability of selective tissue growth in place of implanted biological material) with the increase of metabolic processes providing skin fibroarchitecture restoration.

Absence of balanced growth and differentiation factors specific for skin tissue does not allow for achievement of a pronounced rejuvenating effect and stimulate physiological and reparative regeneration.

There is a known skin care preparation "Garmoniya" [13] containing mammal (human) embryo extract and natural and chemical biologically active additives (geranium oil, coriander oil, plantain tincture, spring birch leaves extract, spring bird cherry leaves extract, aloe and swallowwort latexes, maize oil, propolis extract, palm oil, sea buckthorn oil and hen egg-yolk, glycerine, inorganic salts, such as Hanks' salts, dibasic sodium phosphate, citric acid, with the base to natural and chemical biologically active additives ratio being (0.59 - 5.3):1.0. Embryo extract is produced by homogenization and extraction in saline solution, ultracentrifugation with further supernatant filtration.

The resulting extract contains heteromolecular fractions not characterized by standard physical and chemical methods, water-soluble denatured proteins, peptides, peptidoglycanes, non-standardizable lipoproteins, external application of which is quite immunogenic with a low biological effect.

There is a known method for producing a regenerating skin care cosmetic cream [14] containing, apart from growth factors including interleukine -2, erythropoietin, epidermal growth factor and leucocyte growth hormone, human and/or animal leucocyte metabolites with the following quantitative content of components (weight %): nutrient medium with growth factors and leucocyte metabolites - 0.7 - 8.5; natural and chemical biologically active substances - 7.0 - 27.0, obtained from the nutrient medium in which human and/or animal cells were incubated (e.g., leucocytes), in presence of somatotropin. This composition does not contain cytoplasmic, nuclear and membrane proteins and polypeptides, taking an important signaling part in cell regeneration and repair mechanisms, concentration of growth factors and hormones extracted by leucocytes is not optimal but is their waste and metabolic product, which significantly reduces the composition's biological activity, re-stimulating physiological regeneration and slightly stimulating repair of tissues, not containing tissue-specific factors for skin.

There is a known skin care composition containing the following components: retinoid selected from a group including vitamin A alcohol formulation, vitamin A aldehyde formulation, retinyl acetate, retinyl palmitate and their mixture, oil phase having a low insaturation level, and a stabilizing system selected from a group including at least one oil-soluble antioxidant, chelating agent, licorice extract, hydroquinone, kojic acid, Gatuline A, micromerol, glutathione, magnesium phosphate L-ascorbyl-2, arbutin, placenta extract and their mixes [15].

But the known composition, when applied for a long term, can cause hypertension syndrome and does not make a satisfactory regenerating and rejuvenating effect.

There is a known a skin care preparation based on growing mammal embryos, containing a gelatinous base and components based on growing mammal embryo extract, e.g. bovine embryos [16]. Extraction is made from the throat sweetbread, not from the whole animal embryo. The composition contains (10-30)% of the animal embryo extract and (90-70)% gelatinous base. The preparation is applied to the face and body in order to provide skin cells rejuvenation, wrinkle protection, skin strength enhancement and retardation of tissue aging.

But this cosmetic shows systemic hormonal activity by influencing the entire organism, which has some counterindications, and is not effective enough from the point of view of regenerative action made to the skin due to absence of tissue-specific growth factors and factors preventing from skin insenescence and ageing, preserving and increasing skin elasticity and good condition for a long time.

The closest to the subject group of inventions, including from the point of view of the methods for production of the protein fraction, is the method for producing of a biologically active complex that provides neuroregeneration and neuroprotection [17]. This methods allows for obtaining a mixture of proteins and polypeptides from farm animals' embryo brain taken at a certain gestation period (from the beginning of the middle third to the middle of the last third of pregnancy) by the method of anion-exchange chromatography using for application to the equilibrated column a preliminarily filtered tissue homogenate supernatant with a buffer having pH 7.2 - 8.4; the target fractions are produced by separation of proteins and polypeptides associated with the carrier using 0.05M TRIS Glycine buffer solution as a mobile phase, containing 0.1 mM EDTA and 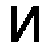 1M NaCl, increasing the concentration by stepwise gradient with a 5% increment. The above complex then undergoes ultrafiltration on a 5 kDa membrane and is desalinated. The target protein/polypeptide fraction characteristics are tested by UV spectrometry, gel-chromatography, denaturing polyacrylamide gel electrophoresis, and amino acid analysis methods. The above biologically active complex has an ultraviolet spectrum absorption peak at wavelength of 280 + 5 nm and a constituent protein concentration from 5 to 110 kDa at least 95% of the total protein content.

Usage of 1M NaCl within the mobile phase at the stage of anion-exchange chromatography for producing this complex generates an excessive ionic strength of the mobile phase equal to 1 mole per litre and inclusion of EDTA and TRIS into the composition increases it still more. In this connection, the obtained target fraction contains a great amount of acidic highly charged proteins and polypeptides, having in brain tissues cells mainly membrane localization performing mainly structural functions and only to a small extent regulatory functions not having tissue-specific nor even expressed biological activity. Further gradient increase of salt concentration with 5% increment still increases the presence of the said proteins, increasing the mixture's biological inactivity, by this reducing its activity. In production of this complex, usage of embryo brain (?) as raw material provides for the presence of tissue-specific proteins, regulating physiological and repair processes of preferentially nerve tissue, producing an unexpressed systemic effect, therefore, the biological activity scope is focused on correction and addressing of nerve system disorders and diseases.

### Disclosure of Invention

The main goal set to the authors was production of a biologically active complex stimulating physiological and reparative regeneration of skin tissue for use in the treatment of skin diseases and traumatic injuries, and a pharmcomposition for use in medicine production in aesthetic medicine and beauty therapy, impacting skin tissue aging and rejuvenescent processes, complying with contemporary international safety requirements to pharmaceuticals and therapeutic cosmetics and quasi drugs.

The objective of this invention is production of a biologically active substance and a pharmcomposition based on it, with enhanced properties and a high biological activity, making skin tissue-specific reparative and regenerative effect for treatment of skin diseases and traumatic injuries, for use in medicine production in aesthetic medicine and beauty therapy, impacting skin tissue aging and rejuvenescent processes, and the method for producing the same by extraction of a biologically active protein-peptide complex and polynucleotides for producing a pharmcomposition from nerve and skin tissues of hoofed farm embryos, which objective is solved through a selected set of procedures and modes. Particularly important was to determine the optimal gestation timing of fetal skin and nervous tissues, as well as a set of reagents, their dosage, extraction and buffering regimes, to maintain effective concentration ratios of proteins and polypeptides fixed evolutionarily in ontogenesis and defining the biological activity of the complex, providing reparative and regenerative tissue-specific effect.

The goal set is achieved by the claimed group of inventions.

The claimed group of inventions includes the protein/polypeptide complex, a method for producing the same from nerve and skin tissues of hoofed farm embryos at a certain gestation stage, and a pharmaceutical composition based on this protein/polypeptide complex.

Now therefore, the goal set is achieved by the new protein/polypeptide complex (PPC), produced from nerve and skin tissues of hoofed farm embryos of gestation stage from the middle of the first third to the middle of the last third of pregnancy, including negatively charged faintly acid, neutral proteins and polypeptides classified as growth and differentiation factors, signaling molecules, determining its biological and pharmacological activity, with molecular weights ranging from 0.5 to 200 kDa, therewith at least 70% of the total protein amount having molecular weights ranging from 20 to 180 kDa, having a specific set of bands at denaturing gel electrophoresis («SDS-Page») in 5% polyacrylamide gel compared to the standard set of marker proteins having molecular weights ranging from 1 kDa to 250 kDa and an absorption peak at wavelength of 215±5 nm, at ultraviolet spectrum recorded at wavelengths ranging from 200 to 500 nm.

The goal set is also achieved with a new method for producing the above PPC in which nerve and skin tissues of hoofed farm embryos of gestation stage from the middle of the first third to the middle of the last third of pregnancy are stepwise:
- homogenized in a buffer with a simultaneous extraction in the presence of reversible proteolysis inhibitors and nonionic detergents and solubilizers at the buffer : tissue ratio of at least 1:0.5, at minimum pH 5.2 and maximum pH 8.5, with further centrifugation at g ranging from 10.000 - 28,000 during 90 - 30 min;
- the resulting supernatant is filtered;
- the filtrate undergoes anion-exchange chromatography, and separating associated proteins using an eluent with ionic strength ranging from 0.14 to 0.21 mmol/L at pH ranging from 5.2 to 8.5, starting to collect target fractions by a mobile phase at ionic strength value of 0.175 mmol/L, increasing it smoothly or stepwise with 0.035 mmol/L increment up to 0.21 mmol/L ionic strength value; the resulting fractions are associated and undergo sterilization filtration.

Another invention of the claimed group is a pharmaceutical composition for use in the treatment of autoimmune, vascular, traumatic, and toxic skin diseases wherein the composition is prepared as a solution containing, as an active ingredient, the protein/polypeptide complex as described abovein concentration of 0.01 - 2.0 mg/ml, produced using the method as described above, polynucleotide fractions in the form of desoxyribonucleic acid sodium salt, obtained from associated ballast tissue material and centrate after the centrifugation of filtered homogenate formed in the course of producing the protein/polypeptide complex using the method under Claim 1 and having absorption peak at wavelength of 260 ± 2 nm and minimum absorption at wavelength of 230 ± 2 nm with ultraviolet spectrum recorded at wavelengths ranging from 190 to 325 nm.

As a pharmaceutically acceptable diluent the pharmaceutical composition may contain a pharmacopoeial buffer and, possibly, excipients including high-molecular compounds, stabilizers, preservatives, and solubilizers.

The pharmaceutical composition may contain, as polynucleotide components, polynucleotides in the form of medium- and low-molecular fraction of desoxyribonucleic acid sodium salt with molecular weights falling, for example, within the range of 12 to 660 kDa (18 - 1000 base pairs), having maximum UV absorption spectrum at wavelength of 260±2 nm and minimum UV absorption spectrum at wavelength of 230±2 nm in the wavelength range from 190 to 325 nm with D_{260/280} ratio from 1.6 to 2.0, presence of indicative specific bands at denaturing gelelectrophoresis in 1.8% agarous gel at ethydium bromide staining, compared to the standard set of marker proteins having molecular weights ranging from 75 to 7,000 base pairs, and blue staining at qualitative ribose test, and an admixture of protein, defined according to Lowry method, not exceeding 0.4 mg/ml (0.17%).

The above pharmaceutical composition is intended, particularly, for external, parenteral (intracutaneous, subcutaneous application) and contact administration to mucous membranes and skin.

Below there is a brief description of the method for producing the complex and the complex itself and the pharmaceutical composition based on it.

### Preferred Embodiment

Now therefore, the protein/polypeptide complex is produced according to the following procedure (some operations are described with detailing not limiting the method):
- nerve and skin tissues of hoofed farm embryos of gestation stage from the middle of the first third to the middle of the last third of pregnancy are defrozen;
- homogenized in a buffer with a simultaneous extraction in presence of reversible proteolysis inhibitors and nonionic detergents at minimum pH=5.2 and maximum pH=8.5, with minimum ratio 1:0.5;
- the homogenate is separated from ballast substances by way of filtering through a close fabric with further centrifugation at g ranging from 10 000 - 28 000 during 90 - 30 min; the resulting ballast tissue material after filtering through a close fabric is combined with centrifuge effluent in order to produce the nucleotide fraction of the pharmcomposition.

### Production of a protein/polypeptide complex:

- The supernatant is filtered through a membrane filter with 0.45 µm pore diameter;
- the filtrate is applied to an equilibrated chromatographic column with an anion-exchange carrier, e.g. Toyopearl DEAE-650M;
- the proteins associated with the carrier are separated by stepwise gradient of the solution ionic strength, using as a mobile phase a buffer solution with maximum ionic strength of 0.14 mmol/l increasing it with a 0.035M increment and starting to collect target fractions using a buffer with ionic strength ranging from 0.175 to 0.21 mmol/l with pH ranging from 5.2 to 8.5;

- the target fractions are associated and diluted with a buffer, e.g. 0.05 M of glycine-NaOH at pH=7.4 to the total protein concentration equal to 1.0 - 2.0 mg/ml;
- the resulting solution undergoes sterilization filtration through a membrane filter with maximum pore diameter 0.22 µm, and the total protein concentration is defined.

All preparatory processes and operations of obtaining the protein/polypeptide fraction are performed at a temperature ranging from +2° to +8°C.

In order to identify the produced protein/polypeptide complex, UV spectrometry, gel-chromatography, and polyacrylamide gel electrophoresis methods are used. The ultraviolet spectrum is recorded at wavelengths ranging from 200 to 500 nm: the absorption peak appears at wavelength of 215 ± 5 nm (Picture 1.). The constituent protein molecular weight is defined by denaturing gel electrophoresis («SDS-Page») in 5% polyacrylamide gel compared to the standard set of marker proteins having molecular weights ranging from 1 kDa to 250 kDa (Picture 2.). The complex has the isoelectric point value ranging from 4.2 to 8.4. The preparation includes proteins with molecular weights ranging from 0.5 to 200 kDa, among which at least 70% have molecular weights ranging from 20 to 180 kDa.

### Production of a polynucleotide fraction for the pharmcomposition and of the pharmcomposition itself:

- associated ballast tissue material and centrifuge effluent of filtered homogenate, are resuspended with a mixer in a solution containing 2.5 mM EDTA and 25 mM sodium citrate with pH 7.0 - 8.0, within the same temperature range in minimum ratio 1:1, incubating the resulting mass for 30 min;
- at the hydrolysis stage, 10M NaOH is added to the resulting mass based on the maximum final alkali molar percentage 2 and, upon stirring, the mixture is incubated for 16 hours at +48°C;
- the mixture is cooled down to +2°C and centrifugated at ∼32,000 g and +4°C for 40 min;
- before supernatant transfer, a thin yellow oil/fatty film is removed from the supernatant surface in centrifugal bottles using filter paper straps;
- 1/10 of 10M NaOH volume is added to purified associated supernatant for second hydrolysis and incubated at +48°C for 5 hours;
- the resulting dihydrolyzate is cooled on ice bath (moist ice, 0°C), then, at continuous agitation and under pH meter control, glacial acetic acid is added to the mixture pH not exceeding 5.4, observing setting of white voluminous sediment of denatured proteins;
- the mixture is centrifugated at ∼32,000 g and +4°C for one hour;
- 0.6 of isopropanol volume is added to the resulting supernatant;
- the resulting mixture, upon incubation at room temperature for 20 min, is centrifugated at ∼32,000 g and +20°C for 1 hour, forming DNA sediment;
- the sediment is diluted in 100 ml H₂O, and 1/10 of 3M sodium acetate volume and 3 ethanol volumes cooled to -20°C are added and agitated;
- the resulting suspension is centrifugated at ∼32,000 g and +4°C for one hour;
- the resulting sediment is diluted in 50 ml H₂O on magnetic stirrer;
- in the final solution upon sterilization filtration through a membrane filter with maximum pore diameter 0.22 µm, and the polynucleotide fraction concentration is defined.

In order to identify the produced pharmcomposition polynucleotide fraction, UV spectrometry and gel electrophoresis in 1.8% agarous gel, as well as qualitative desoxyribose and sodium tests are used. The ultraviolet spectrum is recorded at wavelengths ranging from 190 to 325 nm: the absorption peak appears at wavelength of 260 ± 2 nm (Picture 3). D_{260/280} ratio ranges from 1.6 to 2.0. The constituent nucleotides molecular weight is defined by gel electrophoresis in 1.8% agarous gel at ethydium bromide staining, compared to the standard set of marker proteins having molecular weights ranging from 75 to 7,000 base pairs: the presence of indicative specific bands ranging from 12 to 660 kDa is defined (Picture 4). The qualitative ribose test results in blue staining and an admixture of protein, defined according to Lowry method, not exceeding 18%.

The produced protein/polypeptide complex and nucleotide fraction identification is performed, then, in order to produce the pharmcomposition, they are associated at least in equal parts by concentration with calculation of final total protein and polypeptide concentration in the obtained solution within the range of 0.05 - 2.0 mg/ml at dilution with a pharmaceutically acceptable diluent, e.g. 50mM glycine-phosphate, containing, if necessary, excipients, e.g., with concentrations of 0.5% mannitol, 0.0005% Polysorbate-80 and disodium hydrogen phosphate to pH not below 5.2 and not exceeding 8.5. Repeated identification and filtration sterilization are performed.

The pharmcomposition (PPNC) identifying characteristics are defined by UV spectrometry and gel electrophoresis in 1.8% agarous gel. The ultraviolet spectrum is recorded at wavelengths ranging from 200 to 500 nm: the absorption peak appears at wavelength of 260 ± 2 nm, minimum absorption - at wavelength of 230 ± 2 nm (Picture 5). At electrophoresis in 5% polyacrylamide gel at argentic nitrate staining, the presence of indicative specific protein and nucleotide bands is defined (Picture 6), and at Coomassie brilliant blue staining, only protein fraction characteristic bands are present (Picture 7).

Therefore, for the PPC extraction it is essential:
- to use as the raw material nerve and skin tissues of hoofed farm embryos of gestation stage from the end of the first third to the middle of the last third of pregnancy;
- that detergents and solubilizers were present in the buffer in sufficient concentrations;
- that buffer media pH was falling within the range of 5.2-8.5 at homogenization and anion-exchange chromatography stages;
- that during anion-exchange chromatography the ionic strength (I) of the eluent at the moment of separation of proteins and polypeptides associated with the carrier was not below 0.14 mmol/L, and the target fractions were collected with an eluent with ionic strength not exceeding 0.21 mmol/L;
- that in the described PPC the content of proteins with 20 kDa to 180 kDa molecular weights was at least 70% of the total protein content;
- that while indentifying the claimed PPC and performing denaturing gel electrophoresis («SDS-Page») in 5% polyacrylamide gel compared to the standard set of marker proteins, characteristic bands were present confirming and characterizing the complex protein/polypeptide constituents spectrum. In case of other production parameters, the protein/polypeptide component composition and its constituents ratios will differ.

The claimed biologically active protein/polypeptide complex is fundamentally different from any known biologically active substances produced from animal material by:
- raw material - nerve and skin tissues of farm embryos are used, of a certain gestation stage from the middle of the first third to the middle of the last third of pregnancy;
- methods for production -presence in the extracting (?) buffer detergents and solubilizers and absence of denaturing and degradable compounds - spirits, alkalies, acids, and ferments, allow for obtaining membrane, intercellular, cytoplasmic, nuclear proteins and polypeptides with preserved tertiary structure;
- usage within the mobile phase of millimolar cation concentrations in order to create a moderate ionic strength, by this allowing for obtaining a large amount of nerve tissue specific low charged proteins and polypeptides performing regulatory, signaling, and enzymatic functions, defining a pronounced regenerative and reparative biological tissue-specific activity;
- qualitative composition as a certain fraction of tissue (membranes, cellular, cytoplasmic, nuclear) hydrophilic, low charged proteins and polypeptides, with a concentration ratio of growth and differentiation factors and signaling molecules with molecular weights ranging from 0.5 kDa to 200 kDa, evolutionarily fixed in the ontogenesis;
- identification and standardization of the target fraction obtained - presence of specific bands, characteristic of this complex, in course of electrophoresis in 5% polyacrylamide gel (Picture 6), and removal of molecules with molecular weights exceeding 200 kDa using membrane filtering;
- purity - the resulting protein/polypeptide complex does not contain admixtures such as lipids, peptidoglycanes, lipopolyproteins, carbohydrates, polysaccharides and other compounds;
- administration - parenteral (intradermal, subcutaneous), to mucous membranes (intranasal, subconjunctival) and skin;
- administration safety (absence of toxic, mutagenic, cancerogenic and allergenic effects);
- biological and pharmacological activity (reparative and regenerative) and its specificity (tissue- and organo-specificity).

Hydrolysis parameters change in production of polynucleotide fraction for PPNC pharmcomposition will inevitably result in the pharmcomposition parameters due to polynucleotide components, however, if the recommended concentrations are used, these changes will only insignificantly impact the entire pharmcomposition biological activity. In case of a different raw component for production of the polynucleotide fraction (animal tissues, commercial fish milts, etc.), with production techniques, claimed concentration ratios with the protein/polypeptide complex preserved, and compliance of the resulting fraction with claimed criteria, the biologic activity of the PPNC, as well as that of the pharmcomposition, do not change significantly.

The claimed pharmcomposition in form of PPNC with a pharmaceutically acceptable diluent helps increase the PPC's reparative and regenerative capacity through the specific effect of medium- and low-molecular polynucleotides on antibody-mediated and cell-mediated immunity activation in maximum dosage of 0.1 - 10 mg, and increase the entire composition solution stability and its effect and shelf life.

The invention is illustrated with the following examples without limitation to its scope.

### Example 1. Method for producing the PPC.

### Production of a protein/polypeptide complex from nerve and skin tissues of sheep embryos

440 g of nerve and skin tissues of sheep embryos taken at gestation stage of 16 - 18 weeks is defrozen and homogenated in 1,200 ml of 0.05M TRIS glycine-phosphate buffer solution with pH 8.0, containing 1 mM of EDTA and 0.1% of mannitol in 1:0.5 ratio for 5 minutes at +2°C, the homogenate is separated from ballast substances by filtering through a close fabric with further centrifugation at g=10,000 for 90 min, respectively, associating the ballast tissue material upon filtering through a close fabric with the centrifuge effluent for production of the complex nucleotide fraction.

### So, the methods involves consecutive:

### Production of the protein/polypeptide fraction of the complex:

The supernatant is filtered through a membrane filter with 0.45 µm pore diameter, the filtrate is applied to a 200 ml chromatographic column equilibrated with 4 volumes of 0.05 M glycine-phosphate buffer with pH=5.2, with an anion-exchange carrier Toyopearl DEAE-650M; the proteins associated with the carrier are separated by stepwise gradient, using the same buffer solution containing 0.09 M KCI (I = 0.14 mmol/l), and starting to collect target fractions with salt concentrations ranging from 0.125 M (I = 0.175 mmol/l) to 0.16 M (I = 0.21 mmol/1), increasing them (and, consequently, the ionic strength of the solution) smoothly or stepwise with 0.035 mmol/L (I = 0.035 mmol/L); the resulting fractions are associated in required ratios and diluted with 0.05M glycine-NaOH at pH=5.2 to the total protein concentration equal to 1.4 mg/ml; the resulting solution undergoes sterilization filtration through a membrane filter with maximum pore diameter 0.22 µm, and the total protein concentration is defined.

All preparatory processes and operations of obtaining the protein/polypeptide fraction are performed at a temperature ranging from +2° to +8°C.

The resulting protein/polypeptide complex characteristics:
- the preparation solution ultraviolet spectrum is recorded at wavelengths ranging from 200 to 310 nm: the absorption peak appears at wavelength of 215 ± 5 nm (Picture 1.);
- the constituent protein molecular weight is defined by denaturing gel electrophoresis («SDS-Page») in 5% polyacrylamide gel compared to the standard set of marker proteins having molecular weights ranging from 1 kDa to 250 kDa, the fraction includes proteins with molecular weights ranging from 0.5 to 200 kDa, among which 76% have molecular weights ranging from 20 to 180 kDa (Picture 2);
- the amount of proteins is defined according to Lowry method (without presedimentation) - 1.48 mg/ml.

### Example 2. Method for producing the PPC.

### Production of a protein/polypeptide complex from nerve and skin tissues of pig embryos

300 g of nerve and skin tissues of pig embryos taken at gestation stage of 16 - 18 weeks is defrozen and homogenated in buffer solution with pH=8.5, containing 50 mM of Tris-maleate, 1 mM of (EDTA) and 0.1% of mannitol, for 5 minutes at 8°C. The homogenate is separated from ballast substances by filtering through a close fabric with further centrifugation at g=28,000 for 30 min, respectively, associating the resulting ballast tissue material upon filtering through a close fabric with the centrifuge effluent for production of the complex nucleotide fraction. The supernatant is filtered through a membrane filter with 0.45 µm pore diameter and applied to a 250 ml chromatographic column equilibrated with 4 volumes of maleate buffer with pH=8.5, with an anion-exchange carrier DEAE-Sepharose. The proteins associated with the carrier are separated by smooth gradient of the maleate buffer solution with pH=8.5 containing 0.09M NaCl (I = 0.14 mmol/1), increasing salt concentration to 0.16 M (I = 0.21 mmol/1), starting to collect target fraction with 0.125 M (I = 0.175 mmol/l) salt concentrations. The target fraction is diluted with aspartate-NaOH pH 8.5. The solution undergoes sterilization filtration through a membrane filter with maximum pore diameter 0.22 µm.

In order to characterize the produced PPC, UV spectrometry, gel-chromatography, polyacrylamide gel electrophoresis, and amino acid analysis methods are used. The ultraviolet spectrum is recorded at wavelengths ranging from 200 to 500 nm: the absorption peak appears at wavelength of 215 ± 5 nm (Picture 8.). The constituent protein and polypeptide molecular weights are defined by denaturing gel electrophoresis («SDS-Page») in 5% polyacrylamide gel compared to the standard set of marker proteins having molecular weights ranging from 1 kDa to 250 kDa (Picture 9). The preparation includes proteins with molecular weights ranging from 0.5 to 200 kDa, among which 78% have molecular weights ranging from 20 to 180 kDa. The amount of proteins is defined according to Lowry method - 2.2 mg/ml.

### Example 3. Method for producing the pharmcomposition

*a) Production of the pharmcomposition polynucleotide fraction*: associated ballast tissue material and centrifuge effluent of filtered homogenate weighing 280 g, are resuspended with a mixer in a solution containing 2.5 mM EDTA and 25 mM sodium citrate with pH 7.8, within the same temperature range in volume ratio 1:1.5 (650 ml), incubating the resulting mass for 30 min; 10M NaOH is added to the resulting mass based on the maximum final alkali molar percentage 2 (158 ml) and, upon stirring, the mixture is incubated for 16 hours at +48°C. The mixture is cooled down to +2°C and centrifugated at ∼32,000 g and +4°C for 40 min, removing, before supernatant transfer, a thin yellow oil/fatty film from the supernatant surface in centrifugal bottles using filter paper straps. 1/10 of 10M NaOH volume was added to purified associated supernatant for second hydrolysis and incubated at +48°C for 5 hours. The resulting dihydrolyzate was cooled on ice bath (moist ice, 0°C), then, at continuous agitation and under pH meter control, glacial acetic acid was added to the mixture pH not exceeding 5.4 (63 ml), observing setting of white voluminous sediment of denatured proteins. The mixture was centrifugated at ∼32,000 g and +4°C for one hour; 0.6 of isopropanol volume was added to the resulting supernatant; and, upon incubation at room temperature for 20 min, was centrifugated at ∼32,000 g and +20°C for 1 hour, forming DNA sediment. The sediment was diluted in 100 ml H₂O, and 1/10 of 3M sodium acetate volume and 3 ethanol volumes cooled to -20°C were added and agitated. The resulting suspension was centrifugated at ∼32,000 g and +4°C for one hour, diluting the resulting sediment in 50 ml H₂O on magnetic stirrer. In the final solution, upon sterilization filtration through a membrane filter with maximum pore diameter 0.22 µm, the polynucleotide fraction concentration was defined (3.4 mg/ml).
   The resulting nucleotide fraction characteristics:
   - the solution ultraviolet spectrum was recorded at wavelengths ranging from 190 to 325 nm: the absorption peak appeared at wavelength of 260 ± 2 nm, minimum absorption - at wavelength of 230 ± 2 nm (Picture 8) with D_{260/280} ratio from 1.6 to 2.0 (Picture 3);
   - the constituent nucleotide molecular weight was defined by denaturing SDS-Page electrophoresis in 1.8% agarous gel at ethydium bromide staining compared to the standard set of markers having molecular weights ranging from 75 to 7,000 base pairs. The presence of indicative specific bands within the range of 12 to 660 kDa is noted (Picture 4). At qualitative ribose test, blue staining appears. Protein concentration in the extracted nucleotide fraction, defined according to Lowry method, was 0.3 mg/ml (i.e. ∼0.15% of DNA).
b) The PPC solution produced according to Example 2 is associated with polynucleotide fraction solution presented by a sodium salt of desoxyribonucleic acid produced by a known method described, for instance, in Example 3, under the condition that the final total protein concentration (defined according to Lowry method) and polynucleotide concentration (defined by spectrophotometry), upon dilution with 50 mM of glycine-phosphate buffer with possible addition of excipients, is 2.0 mg/ml. Excipients may be added to the produced PPNC: detergents, solubilizers, preservatives and high-molecular compounds (taking into account the PPNC final concentration), in particular, up to 0.5% mannitol, 0.0005% Polysorbate-80, 0.005% benzalkonium chloride and disodium hydrogen phosphate with pH up to 8.5. The resulting pharmcomposition undergoes sterilization filtration through a membrane filter with 0.1 µm maximum pore diameter, is bottled to aseptic glass bottles and sealed.
   The resulting pharmcomposition characteristics with reference designation of the PPNC complex produced in it:
   - the protein/polypeptide/nucleotide complex identifying characteristics, upon association of produced protein and nucleotide fractions in equal parts, are defined by UV spectrometry and gel electrophoresis in 1.8% agarous gel, the ultraviolet spectrum is recorded at wavelengths ranging from 200 to 500 nm: the absorption peak appears at wavelength of 260 ± 2 nm, minimum absorption - at wavelength of 230 ± 2 nm (Picture 5). At electrophoresis in 5% polyacrylamide gel at argentic nitrate staining, the presence of indicative specific protein and nucleotide bands is defined (Picture 6), and at Coomassie brilliant blue staining, only protein fraction characteristic bands are present (Picture 7).

### Example 4. Method for producing the pharmcomposition

*a) Production of polynucleotide fraction of the pharmcomposition:*
   ballast tissue material and centrifuge effluent of filtered homogenate weighing 175 g, are resuspended with a mixer in a solution containing 2.5 mM EDTA and 25 mM sodium citrate with pH 7.8, within the same temperature range in volume ratio 1:3 (425 ml), incubating the resulting mass for 30 min; 10M NaOH is added to the resulting mass based on the maximum final alkali molar percentage 2 (112 ml) and, upon stirring, the mixture is incubated for 16 hours at +48°C. The mixture is cooled down to +2°C and centrifugated at ∼32,000 g and +4°C for 40 min, removing, before supernatant transfer, a thin yellow oil/fatty film from the supernatant surface in centrifugal bottles using filter paper straps. 1/10 of 10M NaOH volume (42 ml) was added to purified associated supernatant for second hydrolysis and incubated at +48°C for 5 hours. The resulting dihydrolyzate was cooled on ice bath (moist ice, 0°C), then, at continuous agitation and under pH meter control, glacial acetic acid was added to the mixture pH not exceeding 5.4 (48 ml), observing setting of white voluminous sediment of denatured proteins. The mixture was centrifugated at ∼32,000 g and +4°C for one hour; 0.6 of isopropanol volume was added to the resulting supernatant; and, upon incubation at room temperature for 20 min, was centrifugated at ∼32,000 g and +20°C for 1 hour, forming DNA sediment. The sediment was diluted in 100 ml H₂O, and 1/10 of 3M sodium acetate volume and 3 ethanol volumes cooled to - 20°C were added and agitated. The resulting suspension was centrifugated at ∼32,000 g and +4°C for one hour, diluting the resulting sediment in 50 ml H₂O on magnetic stirrer. In the final solution, upon sterilization filtration through a membrane filter with maximum pore diameter 0.22 µm, the polynucleotide fraction concentration was defined (3.4 mg/ml).

   In order to characterize the produced nucleotide fraction, UV spectrometry and gel electrophoresis in 1.8% agarous gel, as well as qualitative desoxyribose and sodium tests were used. The ultraviolet spectrum is recorded at wavelengths ranging from 190 to 325 nm: the absorption peak appears at wavelength of 260 ± 2 nm, minimum absorption - at wavelength of 230 ± 2 nm (Picture 10). D_{260/280} ratio ranges from 1.6 to 2.0. The constituent nucleotides molecular weight is defined by gel electrophoresis in 1.8% agarous gel at ethydium bromide staining, compared to the standard set of marker having molecular weights ranging from 75 to 7,000 base pairs: the presence of indicative specific bands ranging from 12 to 660 kDa is noted (Picture 11). The qualitative ribose test results in blue staining. Protein concentration in the extracted nucleotide fraction, defined according to Lowry method, was 0.28 mg/ml (i.e. ∼0.12% of DNA).
b) The PPC solution produced according to Example 1 is associated with polynucleotide fraction solution presented by a sodium salt of desoxyribonucleic acid produced by a known method described, for instance, in Example 3, under the condition that the final total protein concentration (defined according to Lowry method) and polynucleotide concentration (defined by spectrophotometry), upon dilution with 50 mM of glycine-phosphate buffer is 0.05 mg/ml. Excipients may be added to the produced PPNC: detergents, solubilizers, preservatives and high-molecular compounds (taking into account the PPC and polynucleotides final concentration - 0.05 mg/l of each), in particular, up to 0.5% mannitol, 0.0005% Polysorbate-80, 0.03% Nipagin and disodium hydrogen phosphate with pH up to 5.2. The resulting pharmcomposition undergoes sterilization filtration through a membrane filter with 0.1 µm maximum pore diameter, is bottled to aseptic glass bottles and sealed.

The resulting pharmcomposition with PPNC characteristics:
- the protein/polypeptide/nucleotide complex identifying characteristics, upon association of produced protein and nucleotide fractions in equal parts, are defined by UV spectrometry and gel electrophoresis in 1.8% agarous gel, the ultraviolet spectrum is recorded at wavelengths ranging from 200 to 500 nm: the absorption peak appears at wavelength of 260 ± 2 nm, minimum absorption - at wavelength of 230 ± 2 nm (Picture 12). At electrophoresis in 5% polyacrylamide gel at argentic nitrate staining, the presence of indicative specific protein and nucleotide bands is defined (Picture 13), and at Coomassie brilliant blue staining, only protein fraction characteristic bands are present (Picture 14).

### Example 5. Toxicity Study

The produced pharmcomposition properties were studied using common methods for experimental (non-clinical) study of pharmaceutical substances [18].

Toxicity studies of the original pharmcomposition in acute and subchronic experiments have been carried out. The presentation is a solution with equal protein and polypeptide concentrations of 0.1 mg/ml. The maximal recommended daily dose is 0.4 mg in case of parenteral introduction.

### Experimental Conditions and Research Methods

The study objective was to define the original PPNC pharmcomposition tolerance, toxic, and lethal doses. The experiments were performed in BALB/C mice of both sexes, weighing 18-20 g, at intraperitoneal, subcutaneous, and intracutaneous introductions. The observation time was 14 days. The animals were taken from a nursery with passports. The mice were kept in standard conditions in plastic cages 10 animals in each, 10 in each group. The animals were fed with combined feed, fresh vegetables, farmer cheese. Water and food were freely available to the animals. The vivarium had artificial daylight. The air temperature was +18° - +20°C [19]. The maximum possible PPNC dose for mice is 0.2 ml and it was 10 mg/kg, which exceeded the daily therapeutic dose by 600 times. The animals were withdrawn from the experiment by etherization, dissected and examined for internal organs condition.

### Acute Toxicity Study Results

***Mice.*** The animals weighed 18 to 20 g. The introduced dose was 0.2 ml, which was 10 mg/kg. The number of animals within one group was 10. There were two groups: for intragastric and intraperitoneal introduction. The observation time was 14 days.

*Appearance.* By the end of the experiment, all animals looked healthy. The hair was thick, white. Animals from different groups did not differ in appearance.

*Behavior. The animals' behavior did not differ between the groups and from the norm.*

*Mortality.* In all three groups no fatal cases were recorded.

*Animals' weights.* During the entire observation period six check weighing had been performed. Sharp weight loss or gain had not been recorded.

*Internal organs.* The groups did not differ in internal organs condition. Modification of organs or organomegaly had not been recorded.

### Subacute (Subchronic) Toxicity Study

### Experimental Conditions and Research Methods

The PPNC toxicity study in subchronic experiment was performed in male and female Wistar rats with weights ranging from 200 to 250 g. The animals were taken from a nursery. The rats were kept in standard conditions in plastic cages 5 animals in each. They were fed with pelletized feed, fresh vegetables, farmer cheese. Water and food were freely available to the animals. The vivarium had artificial daylight. The air temperature was +18 - +20°C. Prior to the experiment, the animals were kept in quarantine for 10 days. A group included 10 animals (5 males and 5 females). The groups were divided as follows:
1 group (reference) intact animals
2 group - PPNC T. 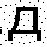 . intracutaneous introduction (π/κ)
3 group - PPNC x 10 π/κ
4 group - PPNC intraperitoneal introduction (B/B) T. 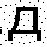 .;
5 group - PPNC B/B x10.

Observation period: one month introduction, one month resorptive effect observation.

Toxicity was assessed against an observation checklist, including:
- survival rate and general appearance: (hair, eyes, ears, limbs, teeth);
- animals' condition and behavior (activity, gait, temperament, food acceptance);
- body weight variation (the rats were weighed prior to and at the end of the experiment);
- physiological functions (breathing, salivation, urination, excretion).

Experimental animals' blood was taken for analysis prior to and at the end of the experiment:
- periferic blood morphological composition (red blood cells, white blood cells, thrombocyte counts, Hb level);
- biochemical values (protein, urea, creatinine, glucose levels), activity of some plasma enzymes (aspartate- and alanine aminotransferase, alkaline phosphatase, lactate-dehydrogenase).

Blood for hematologic study was taken from tail veins. Blood corpuscles were counted using an automatic blood analyzer Picoscale (Hungary). Hb level was defined by cyanmethemoglobin method. Biochemical value levels were measured with automated system. FP-901, «Labsystems» Finland. Upon completion of a chronic experiment, the animals were sacrificed using narcosis overdose for pathomorphological study of organs and tissues. The animals were dissected immediately upon their death, according to a full pathoanatomical scheme. For pathomorphological studies, tissue samples were gelatinated and cryostated to obtain sections up to 5 µm thick. The tissues then were fixed and stained with hematoxylin-eosin. Microscopical investigation was performed with an Opton microscope (Germany). The experimental data were compared to the reference data and analyzed using a statistic software.

### Macroscopic Examination Results

*Survival rate:* all experimental animals survived all introduction methods. Deaths were not recorded neither in experimental nor in reference groups.

*Behavior*: no significant behavior (hyper- or hypoactivity) or condition deviations in experimental animals compared to reference groups were observed. Myotonus was not marked with hypererethism.

*Appearance*: all animals were medium finish, did not suffer from exhaustion or obesity. Hair was smooth and glossy; hairslip or fragility were not detected. Corneal opacity, tearing or any pathological characteristics were not observed. Pinnae were pink with no crust, not inflamed, jerking was not observed. Teeth were of normal color, breaking was not observed.

*Functions:* experimental animals breathing, like that of reference animals, was quiet, of normal rhythm, unobstructed; salivation with no distress; urinary frequency, amount and color of urina fell within physiological range.

Clinical study: *body weight dynamics*: positive in all experimental groups. There was no significant difference between experimental and reference animals. *Haematologic values:* in all groups Hb level, red blood cells, white blood cells, thrombocyte counts fell within physiological range.

*Biochemical study:* the PPNC taken in test dose did not affect the total plasma protein in experimental animals, which is indicative that the preparation being studied does not adversely affect the protein generating liver function. In order to detect possible adversely affect on liver, in course of the chronic experiment, alkaline phosphatase, aspartate and alanine aminotransferase activity was defined in animals' blood plasma.

Data analysis showed no significant changes in the above plasma enzymes activity.

### Pathomorphological data: macroscopic examination

At dissection, in animals of all groups the skin was clear, subcutaneous fat layer is moderate. Internal organs were allocated in a regular way; there were no free fluids in pleural and abdominal cavities. Tracheal and bronchial lumens were free, their mucosae clear, moist, glossy. Lung tissue in the experimental group was more intensely colored than with intact reference animals, but bore no oedema signs. Myocard section showed no pathological findings. The tongue was clear. Oral and esophageal mucosae had no defects. Stomach and bowel bore no traces of irritation. Hepatomegaly was not observed. Renal capsule is easily detachable; medulla and cortical substance were well distinguished at section.

The splenic capsule was smooth, grayish-brown at section, splenic pulp without scraping.

Testes and ovaries were unremarkable. Thymus was grayish, with no hemorrhage.

Thyroid dense with symmetrical portions. Cerebral meninges of moderate blood filling, moist, glossy. Brain substance had a symmetrical pattern at section. Tested animals organs weight indices had no significant difference from reference groups.

Microscopic Study: in all tested groups: in internal organs (liver, kidneys, lungs, heart, spleen, stomach, large and small intestine), internal secretion glands (thyroid, thymus, suprarenal capsules, pancreatic gland), reproductive organs (uterus, ovaries), lymph glands, cerebrum, skin, nose and throat mucosae pathomorphological changes were not detected.

### Subchronic toxicity study in immature animals at intracutaneous introduction in immature animals.

Study was performed in three weeks old baby chinchilla rabbits weighing 300 to 500 g. The preparation was introduced once and twice daily by 0.2 ml of 0.1% solution into 5 points for two weeks. The animals were taken from a nursery. The rabbits were kept in standard cages 1 animal in each. They were fed with pelletized feed and fresh vegetables. Water and food were freely available to the animals. The vivarium had artificial daylight. The air temperature was +18 - +20° C. There were 3 groups, 5 animals in each. The groups were divided as follows: 1 group (therapeutic dose) - PPNC 0.1 mg/ml 0.2 ml introduced to 5 points once daily; 2 group - PPNC 0.1 mg/ml 0.2 ml introduced to 5 points twice daily; 3 group - intact animals. Introduction period was 2 weeks.

Toxicity was assessed against an observation checklist: by survival rate, general appearance, animals' condition and behavior, body weight variation (the rabbits were weighed at the beginning and at the end of the experiment). In course of the experiment, the local irritant effect was defined by changes at visual and histological examination in skin, at sites of injection.

Prior to and at the end of the experiment, experimental animals were subjected to periferic blood morphological composition analysis (red blood cells, white blood cells, thrombocyte counts, Hb level); biochemical values (protein, urea, creatinine, glucose, total cholesterol and triglycerides levels), activity of some plasma enzymes (aspartate- and alanine aminotransferase, alkaline phosphatase, lactate-dehydrogenase). To define the said values, blood was taken from auricular veins in the amount of 1.5-2.0 ml. Blood corpuscles were counted using an automatic blood analyzer Picoscale (Hungary). Hb level was defined by hemiglobincyanide method. Biochemical value levels were measured with automated system. FP-901, «Labsystems» Finland.

Upon completion of a chronic experiment, the animals were sacrificed using narcosis overdose for pathomorphological study of organs and tissues. The animals were dissected immediately upon their death, according to a full pathoanatomical scheme.

Morphometric organs parameters were estimated using Sartorius scales (Germany) with further calculation of organs weights and their standard deviations.

For pathomorphological studies, fresh tissue samples were gelatinated and cryostated to obtain sections up to 5 µm thick. The tissues then were fixed and stained with hematoxylin-eosin. Microscopical investigation was performed with an Opton microscope (Germany).

The experimental data were compared to the reference data and analyzed using statistic software.

### Examination Results

### Local Irritative Effect Estimate

### In-life biomicroscopic study

During the entire observation period the animals preserved normal behavior; hyperemia, oedema, and other changes at sites of injection were not detected.

*Reference intact group.* Skin covers at sites of injection were rose-pink, without oedemas and hyperemia; hair of normal thickness, with healthy gloss without excessive sweat and oil secretion. During observation, defects were not found on epidermis. Reflexes fell within physiological range.

Confocal microscopy: epidermic layer and derma itself had a densely packed structure of terminally differentiated cells. Cells have gap junctions.

*In the therapeutic dose group.* Skin covers at sites of injection were rose-pink, without oedemas and hyperemia; hair of normal thickness, with healthy gloss without excessive sweat and oil secretion. During observation, defects were not found on epidermis. Reflexes fell within physiological range. Confocal microscopy: epidermic layer and derma itself had a densely packed structure of terminally differentiated cells. Cells have gap junctions. No pathological changes were detected.

*In group x10.* No pathological skin changes were detected.

### Clinical and Biochemical studies

Blood analysis: haemoglobin contents, red blood cells, white blood cells, thrombocyte counts in all groups fell within physiological range. Experimental groups data had no significant difference from reference intact groups data and that of the reference substance.

*Biochemical study*: blood glucose, alanine and asparaginic aminotransferase activity in all groups fell within physiological range.

**Conclusion.** Toxicity study of the PPNC in subchronic experiment was performed to immature animals.

Intracutaneous introduction of PPNC pharmcomposition both in therapeutic and tenfold dose during two weeks did not result in internal organs changes, nor had a toxic effect on hemic system, nor local irritative effect.

### Example 6. Mutagenicity of the PPC

The following set of tests was used to assess mutagenic properties of the claimed protein/polypeptide complex [20]:
- gene mutation account in Ames test of Salmonella / microsome using Salmonella typhimurium TA 97, TA 98 and TA 100 strains as test objects;
- micronuclei account in mice marrow cells.

A sample of protein/polypeptide complex was tested, which was transparent liquid, bottled in a sterile dark glass bottle in the amount of 20 ml, containing the substance in 0.1 mg/ml concentration.

### The protein/polypeptide complex mutagenic properties assessment in Ames test.

Mutational test on Salmonella typhimurium is a bacterial test system for account of gene mutation to histidine prototrophy when subjected to chemical compounds and/or their metabolites, inducing base substitution or reading frame shift mutations in this organism genome. Mutagens inducing base pair substitution are agents inducing base pair substitution mutations in DNA molecule. Mutagens inducing genetic code reading frame shift mutations are agents inducing excess or deficiency of single or multiple base pairs in DNA molecule.

This method is used to detect the ability of pharmaceutical substances or their metabolites to induce gene mutations in Salmonella typhimurium indicator strains. The bacteria are treated with the compound being tested with metabolic activation system (CM+) or without metabolic activation (CM-). Upon incubation for a certain period, the number of revertant is counted in different test strains in comparison to the number of spontaneous revertants in negative control options (untreated cultures or solvent treated cultures). If a compound and/or its metabolites being tested are mutagenically active, they will induce back mutations from auxotrophy to histidine prototrophy in histidine-dependent Salmonella typhimurium strains.

For testing, a set of Salmonella typhimurium indicator strains was used, allowing for recording reading frame shift (TA 98 and TA 97) and base substitution (TA 100) mutations.

Strains were taken from Russian National Collection of Industrial Microorganisms of Research Institute for Genetics and Selection of Industrial Microorganisms («Genetika»).

The procedure for handling bacterial cultures, strain genotype check, regulations for their museum storage, necessary experimental equipment, labware, chemical agents, nutritional media and solutions, preparation of rat liver homogenate and activating mixture, and findings statistical analysis methods complied with standards as described in reference literature.

For metabolic activation, the S9 Wistar male rats liver fraction was used; 5 days prior to sacrifice the rats were subjected to microsomal enzymes inducer introduction - sovol (300 mg/kg, single dose, intraperitoneally). In order to monitor the metabolic activation system, ethidium bromide was used (10 µg/dish) on TA 98 strain at CM+.

The PPC in form of sterile solution containing 0.1 mg/ml total protein was studied: 1.0 and 0.3 ml of original solution per dish and three tenfold dilutions in physiological solution (0.1ml/dish). The substance test doses were 300; 100; 10; 1 and 0.1µg/dish.

The experiment was supported with positive controls using substances inducing mutations in corresponding tester strains, with and without activation conditions. In cases without activation, sodium azide was used in the amount of 10 µg/dish for TA 100 strain at CM-; 2,7- diamino -4,9- dioxy -5,10- dioxo-4,5,9,10-tetrahydro-4,9- diazopyrene (DIAM) - µg/dish for TA 98 strain at CM-; 9-aminoacridine (9AA) - 50 µg/dish for TA 97 strain at CM-. In order to monitor the metabolic activation system, ethidium bromide was used - µg/dish on TA 98 strain at CM+. As a negative control, physiological solution was used (0.1 ml per dish).

Selective semi-enriched agar (0.7%) in test tubes was melted on boiling-water bath at 100° C and placed to a temperature-controlled water bath at +45 - +46° C.

First, to test tubes with agar 0.1 ml of sample was applied in required dilution concentration, then - 0.1 ml of bacteria suspension and 0.5 ml of microsomal activating mixture (for metabolic activation option). Then the tube content was quickly stirred and plated out to the lower minimum agar layer in Petri dish. Duration of microsomal activating mixture application and semisolid agar plating onto the dish did not exceed 10-15 seconds. The dishes were left at room temperature for 30-40 minutes and after full solidifying were placed to a thermostat at +37°C. The results were recorded after 48 hours of incubation.

Concurrently, the experiment included options without a metabolic activation system (CM-) and with a metabolic activation system (CM+). In the CM- option, direct mutagens effect can be recorded due to the original substance structure activity. Meanwhile, the effect of promutagens, i.e. compounds whose effect is related to formation of mutagenic metabolites, can be taken into account in comparing CM- and CM+ options of the substance study analysis findings. In each reference and experimental option 2 dishes were used. The mutagenic effect was considered significant if the average number of revertant colonies per dish in the experiment option twice and more exceeded that of the reference option. The experiment results were taken into account provided there was a standard response in all options of positive and negative control. The number of revertant colonies in control with solvent in options CM- and CM+ fell within the range of spontaneous level variations for these strains. The strains response to standard mutagens fell within the range of ordinary levels.

The studied PPC in all concentrations tested showed no mutagenic effect on TA 100, TA 98 and TA 97 strains in options with and without metabolic activation system.

CONCLUSION: the protein/polypeptide complex does not induce gene mutations in Salmonella typhimurium test strains within the entire range of tested concentrations.

### The PPC mutagenic properties assessment by micronucleus method in mammalian cells.

Cytogenetic activity is the ability of a substance to cause structural and numeral chromosomal distortions in somatic and germ cells.

Micronuclei are small DNA-containing formations consisting of acentric chromosome fragments or ana-telophase laggards. At telophase stage, these fragments can be incorporated into daughter cell nuclei or form single or multiple micronuclei in cytoplasm.

The study objective was detection and quantitative estimate of cytogenetic (mutagenic) activity of pharmacologic substances in polychromatophil cells (PCC) of mammal marrow. The method is based on microscopic recording of cells with micronuclei.

The experiments were performed to 2 months old male and female F1 (CBAx C57BI6/J) cross-breed mice weighing 18-20 g. Each group included 6 animals. The animals were kept at 12 hours light pattern with free food and water access. Animal experiments for assessment of mutagenic properties of the protein/polypeptide complex with single and five-time dosing were conducted in compliance with official records. Three sessions of experiments were carried out with corresponding controls: therapeutic dose was tested on male animals with a single introduction; subtoxic dose was tested on male animals with a single introduction; therapeutic dose was tested on male animals with a single introduction; subtoxic dose was tested on male animals and female with five-time introduction.

Taking into account that the possible presumed method for usage of the PPC at a clinic was intracutaneous, subcutaneous introduction, and external application, in this study subcutaneous introduction of the substance to mice was used. The PPC complex dosing for assessment of mutagenic activity was calculated based on the maximum human recommended daily therapeutic dose (TD). Subcutaneous introduction of PPC was recommended in the amount of 0.2 to 6 ml at 0.1 mg/ml concentration. In this case, a human therapeutic dose (TD), including a child's dose, can be 0.08 mg/kg/daily. In order to calculate an experiment dosing for mice, a conversion factor is recommended that takes into account a human or animal body surface area to weight ratio. In our study, it was 8.33. Therefore, the mice TD was approximately 8.33 x human TD (0.7 mg/kg).

As a subtoxic dose, 10mg/kg, the maximum possible in this experiment conditions dosing was used, as the substance was presented in 0.1 mg/ml concentration, and the normally used amount of substance for mice is 0.1 ml per 10 g. Respectively, in conversion to humans, the maximum approved dosing is 1.7mg/kg.

Seven animal groups were formed: four experimental groups and three corresponding reference groups. The substance in the amount of 0.1 mg/kg was introduced five times at 24 hours interval to males and females. All animals were sacrificed 6 hours upon the last introduction of the PPC.

In two other experimental groups, the PPC was introduced in single dosing to males in doses of 0.2 mg/kg and 0.5 mg/kg.

Normal saline solvent was used as a negative control. It was introduced subcutaneously to male and female mice of two reference groups for the same period and in the same amount as to the experimental animals. As a positive control, 20 mg/kg cyclophosphamide was used, diluted ex tempore in normal saline at a single intraperitoneal introduction 24 hours before sacrifice.

Marrow cell preparations for micronuclei count were made by a standard method in compliance with guidelines «O 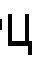 eHκa MyTa 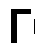 eHHO 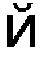 aκT 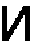 BHOCT 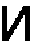 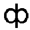 aκTOpOB Oκpy 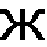 a 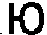 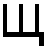 e 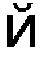 cpe 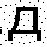 bI B κ 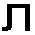 eTκax pa3HbIx op 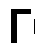 aHOB M 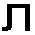 eκO 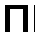 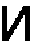 Ta 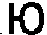 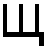 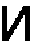 x M 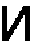 κpO 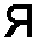 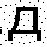 epHbIM MeTO 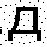 OM» ("Environmental Factors Mutagenic Activity

Assessment in Various Mammal Organs Cells by the Micronucleus Method"). The animals were sacrificed through cervical dislocation 6 hours after the last introduction. Both thigh bones were detached and muscles were removed from them. In order to wash out the marrow, fetal calf serum was used (produced by NPP PanEko). The marrow from both thigh bones was washed out into a microtube using a syringe. The suspension was centrifugated at 1,000 rpm for 5 min, the supernatant was removed, and the sediment was carefully resuspended. A smear was prepared from a drop of the suspension, which was further air dried out. Then it was fixed with methanol for 3 minutes. Stained according to Romanowsky-Giemsa method. 2,000 polychromatophil cells from each animal were analyzed in encoded preparations. At counting 500 red blood cells, polychromatophilic to normochromatic erythrocytes ratio was defined. Upon analysis completion, the preparations were decoded.

The positive result criterion is the replicable and probably significant increase of micronuclei polychromatophilic erythrocytes number at least in one group compared to the reference group. Achievement of a positive result is indicative of the fact that the substance induces chromosome damage and/or cell mitotic apparatus disturbances in experimental animals.

Micronuclei count statistical analysis was performed by comparing experimental series to reference once by X2 criterion; intergroup comparison of polychromatophilic erythrocytes share was carried out using Mann-Whitney test.

Findings of micronuclei count in mice medulla polychromatophilic erythrocytes: in neither option of the experiment the PNC caused increased frequency of micronucleus cells in mice medulla when comparing experimental and corresponding reference series. Cyclophosphamide (positive control) introduced subcutaneously to male mice in 20 mg/kg dosage caused increased frequency of micronucleus cells by 19.6 times (62.1 % against 3.16 % in reference, P<0.001). Polychromatophilic erythrocytes share in the total medulla erythrocytes was approximately on the same level in experimental and reference series groups, which is indicative of absence of the composition toxic effect on hematogenesis in tested dosing. During application of positive control (cyclophosphamide), polychromatophilic to normochromatic erythrocytes ratio was found to shift to the latter (compared to control P<0.005).

Therefore, the PPC mutagenic activity was not detected in mice medulla polychromatophilic erythrocytes micronuclei induction test at single and five-time dosings introduced subcutaneously to male and female mice in 0.7 mg/kg dosage, which in conversion is equivalent to a human therapeutic dose. Also, the mutagenic activity was not detected at single introduction of the PPC to males in maximum possible dosing - 10 mg/kg. The PPC did not show toxic effect by the "polychromatophilic erythrocytes share" criterion.

### Conclusion on the PPC mutagenic properties assessment

The protein/polypeptide complex does not make a mutagenic effect in Ames test of Salmonella / microsome and in mice medulla polychromatophilic erythrocytes micronuclei induction test in experiments performed in compliance with the Guidelines for Experimental (Non-Clinical) Study of New Pharmaceutical Substances.

### Example 7. Biological Activity Study

**Reparative effect.** This type of pharmacological effect was established in a standard model with a full thickness skin defect in mice who were subjected to the claimed PPC injections made around the defect area in 0.2 - 1.0 mg/kg dosing. Under the action of the preparation, the healing time reduced from 17 to 10 days (reference healing time). A similar effect is made by Amniocenum, a preparation produced from placenta and introduced in 75 mg/kg dosing. In this respect, a substantial advantage of the claimed PPC is absence of scars in course of healing.

**Adaptogenic effect.** This type of effect in the process of development of new drugs is becoming one of the most vital areas of research, as it can provide the ability to influence physiological processes at physical, thermal, chemical overloads, especially for avoidance of poisoning, burns, for tolerance enhancement, etc. In course of the claimed PPC testing, it was established that a preliminary treatment of an animal (mouse) for 7 days in a therapeutic dose (0.1 ml/kg) prevents animals from dying of a toxic dose of noxious substances (strychnine), lethal exposure, infecting with blue pus bacillus.

**Stress-relieving effect.** The antistress effect of the claimed PPC was studied on the basis of multivariable techniques for assessment of condition of different components of emotional stress tolerance in rats (behavioral and visceral components). These studies showed that the claimed PPC can increase emotional stress tolerance, i.e. prevent from higher nervous activity and systemic immunity functional disorders and dysmetabolisms of cardiac function. The most effective dosage was 20 µl per 100 g of a rat weight, which corresponds to 2 ml per 1 injection for a human.

**Anticoagulative effect.** In course of study of the claimed PPC impact on blood coagulability it was established that in case of introduction of 0.2 mg/kg during one month, the PPC shows ah anticoagulative effect which disappears 30 days after termination of administration. This fact can be of significant practical importance in treatment of conditions with symptomatic hypercoagulability.

### Example 8. Burn-treating and Reparative Effect.

20 F₁ (AMCY x Wistar) rats weighing 250-270 g were anesthetized by intraperitoneal introduction of 60 mg/kg thiopental. In order to make a burn, on a 3x3 cm depilated area in the scapular region a 1cm² four-layered surgical gauze wipe moistened with distilled water was applied, and a 1cm² cautery electrode heated to 200°C was pressed.

The burn degree was regulated by the time of electrode exposure which was 3 to 15 seconds.

The rats were sacrificed on the 3, 7, 14, 23, 30 and 60^{th} day upon making a burn; the burnt tissues were subjected to histological examination. Macroscopic observation showed that immediately after the burn, a sharply marginated dry, dense, off white-grey, with patches of cyanotic color eschar appears on skin. The skin around the thermal influence area becomes oedematous. A pink crown appears around the eschar.

10 rats of the PPNC pharmcomposition group received intracutaneous injection in 8 points around the periphery of the burn in the aggregate dose of 0.2 mg/kg, which was 2 ml of the solution. The rest 10 rats formed the reference group receiving intracutaneously 2ml of physiological solution in the same amount of points.

On the third day upon making a burn, a vibrant cell response to the damage appears in the form of lymphomonocytic infiltration around the damaged and unaffected tissues boundary.

On the seventh day, in both groups granulation tissue is found under the eschar, moderately infiltrated with monocytes and lymphocytes, but the experimental group differed from the reference group by the fact that, apart from the ordinary granulation tissue appearing on the damaged area, healing collagen fiber, though thin and delicate, are already aligned with reticular dermis histoarchitecture, and damaged tissues of hypoderm and muscular layer were replaced with loose connective tissue with a slight fibroblast excess. From the boundaries inwards, epidermis was growing under the eschar, with the skin layer structure under the epidermis not different from the reference group. Subepidermic capillaries were enlarged and sanguine in both groups.

By the 14^{th} day, in the PPNC group, under the thin dense eschar and the following narrow area of cell debris, new connective tissue was found, in which thin and weakly fuchsinophil collagen fiber bundles clearly reproduced the reticular dermis collagen fiber bundles structure and architectonics. Under the regenerating derma, there is tissue consisting of a delicate mesh of reticular fibers, between which fat cells are found. This allows to speak of subcutaneous fat structure recovery. Within the reference group, some chaotic nature of collagen fiber bundles arrangement without clear architectonics was observed, under which there was a mesh of reticular fibers with collagen fiber bundles interwoven.

On the 23^{rd} day, in both groups the damaged surface was fully epithelized. Epidermis, slightly thickened on correctly located margins, formed pectiniform outgrowths into subjacent tissues. In the PPNC group, the papillary dermis had closer packed fibers and an increased cellularity compared to the reference group.

On the 30^{th} day of the experiment, further derma collagen fiber maturation was observed in both groups, and in the PPNC group, the fibers were slightly smaller with a pronounced structuring. In all animals of the reference group, a significant epidermis thickening was observed.

On the 60^{th} day, the thermal burn area examination showed no visual difference from the subjacent tissues in the PPNC group, there was no qualitative morphological change compared to normal skin tissue. In the reference group, hypertrophic cicatrization was observed with a protuberance over the subjacent skin cover. Morphological examination showed increased content of destructured collagen in all skin layers.

### Example 9. Immunomodulatory activity

Immunomodulatory properties of the PPNC pharmcomposition produced by the same method as in Examples 3 and 4 were determined by macrophage activation of Wister rats peritoneal exudate in experiments in vitro on enhancement of the enzyme activity in reduction of nitro-blue tetrazolium to diphormazane (NBT-test) and on enhancement of the phagocytic activity to E.Coli.

Into the abdominal cavity of decapitated animals 20 ml of colorless Hanks solution containing 20 U / ml heparin was injected by a syringe with a needle. After a 2-3 minute abdomen massage, the introduced liquid was syringed out of the cavity with the same syringe through an incision, which liquid, after filtration through a nylon filter, was centrifuged at g=150-200 for 15 min. The resulting pellet was suspended in a half volume of a fresh portion of Hanks solution and centrifuged again under the same conditions. Washing of the extracted cells was carried out for 3 - 4 times, after which the cell concentration in the suspension was adjusted to 80 x 10⁶ cells / ml. Macrophage content in tissue exudate of all cells was 25-35%. Determination of the enzymatic activity of rat peritoneal exudate macrophages was defined by spectrophotometry using reduction of the nitro blue tetrazolium into diphormazane. The cell sediment of rat peritoneal exudate was diluted with colorless Hanks solution to 80 x 10⁶ cells / ml concentration. To the cell suspension, 50 µl sample with pH=7.4 and the PPNC concentration of 0.1 mg / ml was added. The mixture was incubated on water bath at +37 ° C with constant shaking for 30 min, then 50 µl of nitro blue tetrazolium by Reanal (Hungary) solution was added, saturated at 20° C, and incubation was continued for another 30 min. Then 3 ml of acetone was added and centrifuged at 2,000 g for 20 min. The supernatant acetone extract was scanned photometrically at a wavelength of 515 nm. As a control for comparison of enzymatic activity, a sample with a saline-based preservative was used. Phagocytic activity of rat peritoneal exudate cells was determined by their ability to capture the E.Coli. To 50 µl of cell suspension was added 50µl sample with pH=7.4 and the preparation with the total protein concentration of 0.05 mg / ml was added to 50 µl of the cell suspension. The mixture was incubated on water bath at +37 °C with constant shaking for 30 min. Upon incubation, the mixture was suspended in 10 +ml of colorless Hanks solution at +4 °C and centrifuged at 150-200 g for 15 min. The cell sediment of rat peritoneal exudate was diluted with Hanks solution to a concentration of 2.5 x 10⁶ cells / ml. The resulting slurry in the amount of 0.2 ml was applied to an 18 x 18 mm piece of glass and incubated at +37 ° C for 30 min in an atmosphere containing 5% carbon dioxide (CO₂ for attaching macrophages to the glass. Upon incubation, for removal of unattached cells from the glass, the latter was rinsed three times in cups containing Hanks medium. To the cells remaining on the glass, 0.2 ml of E.Coli suspension with concentration of 20 x 10⁶ cells / ml was added and incubated under the same conditions for 45min. Subsequently, the glass was rinsed again three times, fixed with methanol and stained according to Romanowsky-Giemsa method. Phagocytosis index (PI) was determined using the following formula: PI (0 + 2,5 n + 6T + 9p + 10P) / number of cells with 0 - the number of cells not phagocytizing E.Coli; n - the number of cells that have phagocytized 1-4 E.Coli cells; T - the number of cells that have phagocytized 5-7 E.Coli cells, P - the number of cells that have phagocytized 8-9 E.Coli cells; R - number of cells that have phagocytized more than 10 E.Coli cells. Macrophages were identified by non-specific esterase staining.

Wister rats peritoneal exudate macrophage activation level in experiments in vitro in the PPNC group was significantly exceeding that of the reference group with p <0.05 (12 times) in study of enzyme activity increase in reduction of nitro-blue tetrazolium into diphormazane (NBT-test) and 8 times (p <0.01) in increase of phagocytic activity for E.Coli.

### Example 10. Regenerative Activity and Rejuvenescent Effect

Patient L., 41 y.o. The woman applied in autumn complaining of expressed old wrinkles and appearance of new ones after a vacation spent in the South. Examination revealed crow's feet surface wrinkles in lateral angles of eyes, nasal bridge, forehead, expressiveness of nasal labial fold. The face skin is dry and thinned, its turgor is decreased. Expressed grayness and thinness of hair catch the eye. 5 treatment sessions have been run during three weeks with 3 days intervals, consisting of intracutaneous injections of the claimed PPC according to Example 1 and the pharmcomposition produced according to Example 3 taken in concentration of 0.1 mg/ml and in the dose of 0.4 mg per session. The preparation was introduced intracutaneously, symmetrically, directly into the wrinkle lines in 10 points on each side, 0.2 ml per point. Injections were made symmetrically chequerwise, at equal intervals from each other. As early as the end of the first week, the following results were noted: the face skin acquired a fresh natural look; its tonicity and elasticity were restored. The external canthus lines had virtually disappeared and on the forehead and nasal bridge have significantly smoothed. By the end of the third week, gray hair amount has decreased. The result over the coming months was stable; the patient was satisfied with the treatment, remarking a significant decrease of gray hair and increase of hair thickness. Therefore, the proposed pharmcomposition with PPNC allows for improving the efficiency of skin aging treatment due to quick restoration of its fibroarchitecture and systemic immunoregulatory effect on the entire organism.

**Example 11.** Patient 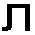 ,.62 y.o. Diagnosis: recurrent parietofrontal basalioma with a pronounced postsurgery wound defect and a long-run epithelialization failure. The patient had an up to 80 cm² visual wound skin defect with occasional sagged granulations, signs of inflammation with suppuration areas. The patient has been suffering th disease for 12 years with a constant recurrence of the neoplastic process. This defect appeared three years ago after one of removals of the recurrent tumor by the cold plasma method, after which, regardless of all attempts to cover it (skin grafting 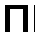 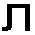 acT 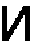 κa, skin transplantation, medication), the wound didn't epithelize. The PPNC pharmcomposition was administered in course doses subcutaneously in 0,1 ml of 0.1% solution to 10 points for 5 days with further external application of the preparation in the form of a water-soluble ointment base in 0.1 mg/ml concentration, together with antibiotics (Levomikol ointment) for ten days once daily. By the end of the first week, the wound had cleared, typical granulation tissue had appeared. By the end of the course, the wound had fully epithelized, occasional small (up to 1-1.5 cm²) areas (4) with a crust. Upon debridement of the wound in a week and a 7 days course of the PPNC external application, the wound fully epithelized. Absence of a characteristic prominent skin tissue scar was observed.

The attached pictures 1-14 show key characteristics of the protein-polypeptide complex, nucleotide fraction, and the protein/polypeptide/nucleotide complex, the pharmcomposition is based on.
Picture 1 shows the Example 1 protein-polypeptide complex solution UV-spectrum in the range of 200 to 310 nm wavelengths, with the absorption peak observed at wavelength of ∼(215±5) nm;
Picture 2 shows denaturing gel electrophoresis of the Example 1 protein-polypeptide fraction solution (2) in 5% polyacrylamide gel with Coomassie brilliant blue staining compared to the standard set of marker proteins having molecular weights ranging from 1 kDa to 250 kDa;
Picture 3 shows the Example 3 pharmcomposition nucleotide fraction UV-spectrum, recorded in the range of 190 to 235 nm wavelengths: the absorption peak is observed at wavelength of ∼(260±2) nm, minimum UV absorption spectrum at wavelength of (230±2);
Picture 4 shows the complex nucleotide fraction (2) electrophoresis in 1.8% agarous gel at ethydium bromide staining, compared to the standard set of markers having molecular weights ranging from 75 to 7,000 base pairs. The presence of indicative specific bands ranging from 12 to 660 kDa is defined.
Picture 5 shows the PPNC pharmcomposition solution UV-spectrum in the range of 200 to 310 nm wavelengths with the absorption peak at wavelength of ∼(260±2) nm and minimum UV absorption spectrum at wavelength of (230±2).
Picture 6 shows the Example 3 PPNC pharmcomposition (1) denaturing gel electrophoresis in 5% polyacrylamide gel compared to the standard set of marker proteins having molecular weights ranging from 1 kDa to 250 kDa (2) at argentic nitrate staining. The presence of indicative specific protein and nucleotide fraction bands is defined.
Picture 7 shows the Example 3 PPNC pharmcomposition (1) denaturing gel electrophoresis in 5% polyacrylamide gel compared to the standard set of marker proteins having molecular weights ranging from 1 kDa to 250 kDa (2) at Coomassie brilliant blue staining (2).
   The PPNC protein fraction characteristic bands are present.
Picture 8 shows the Example 2 protein-polypeptide complex solution UV-spectrum in the range of 200 to 500 nm wavelengths, with the absorption peak observed at wavelength of ∼(215±5) nm.
Picture 9 shows the Example 2 protein-polypeptide complex (2) denaturing gel electrophoresis in 5% polyacrylamide gel at Coomassie brilliant blue staining, compared to the standard set of marker proteins having molecular weights ranging from 1 kDa to 250 kDa (1).
Picture 10 shows the Example 4 pharmcomposition nucleotide fraction UV-spectrum, recorded in the range of 190 to 235 nm wavelengths: the absorption peak is observed at wavelength of ∼(260±2) nm, minimum UV absorption spectrum at wavelength of (230±2) nm.
Picture 11 shows the Example 4 pharmcomposition nucleotide fraction electrophoresis in 1.8% agarous gel at ethydium bromide staining, compared to the standard set of markers having molecular weights ranging from 75 to 7,000 base pairs. The presence of indicative specific bands ranging from 12 to 660 kDa is defined.
Picture 12 shows the Example 4 PPNC pharmcomposition solution UV-spectrum in the range of 200 to 500 nm wavelengths with the absorption peak at wavelength of ∼(260±2) nm and minimum UV absorption spectrum at wavelength of (230±2) nm;
Picture 13 shows the Example 4 PPNC pharmcomposition (2) denaturing gel electrophoresis in 5% polyacrylamide gel compared to the standard set of marker proteins having molecular weights ranging from 1 kDa to 250 kDa (1) at argentic nitrate staining. The presence of indicative specific protein and nucleotide fraction bands is defined.
Picture 14 shows the Example 4 PPNC pharmcomposition (2) denaturing gel electrophoresis in 5% polyacrylamide gel compared to the standard set of marker proteins having molecular weights ranging from 1 kDa to 250 kDa at Coomassie brilliant blue staining. The PPNC protein fraction characteristic bands are present.

### Industrial Applicability

The inventions allow for creation of a biologically active protein-polypeptide complex stimulating physiological and reparative regeneration of skin tissue for treatment of skin diseases and traumatic injuries, and for use in medicine production in aesthetic medicine and beauty therapy, impacting skin tissue aging and rejuvenescent processes. An effective natural pharmaceutic composition containing a protein/polypeptide/nucleotide complex has been created, complying with contemporary safety requirements to pharmaceuticals and cosmetics.

Sources of information taken into account:
1. RF Patent No.2229886 C1
2. EA No.000633 B1
3. RF Patent No.2237486 C1
4. UA Patent No.15241, IPC 6 A 61 K 35/50, claim publication date 15.09.2000. Bul. No.4, 2000 /.
5. « 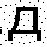 ep 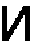 HaT» κOM 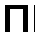 aH 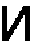 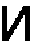 «TexHOMe 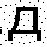 cepB 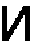 c» 3AO Φ 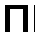 (Pocc 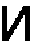 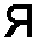 ) ( 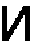 H 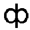 OpMa 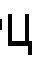 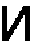 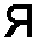 O 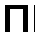 y 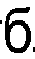 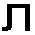 κOBaHa C 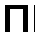 paOBO 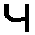 H 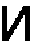 κ «B 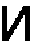 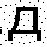 A 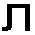 b» MOCκBa, «ACTpaΦapMCepB 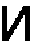 C» 2010 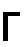 ., 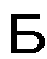 -506). (Derinat produced by ZAO Pharmaceutical Company "Technomedservis" (Russia) (the information was published in "Vidal" Drug Reference Book, Moscow, AstraPharmService, 2010, B-506))
6. RF Patent No.2235550, IPC A61 K35/48, A61K35/50
7. RF Patent No.2400240, IPC A61K35/50, A61P15/00, A61P17/02
8. RF Patent No.2228759, IPC A6K1 35/50
9. RF Patent No.2041717, IPC A6K1 35/55
10. RF Patent No.2119790, IPC A 61 K7/00, publ. 10.10.1998
11. Patent No. RU 2289417 C1
12. RF Patent No.2189257, I PC A 61 L 27/00, A 01 N 1/00, 20.09.2002
13. RF Patent No.2142784 IPC A61K7/48, A61 K35/78
14. RF Patent No.2144815, C1 (51) A61K7/00, A61 K7/48, A61P17/16
15. US Patent No.9703169
16. Application of France No. 2530466, IPC A 61 K 7/48, 27.01.2084
17. International Application Nº WO 2009/088314, 2007
18. PyκOBO 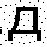 CTBO no 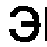 κC 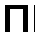 ep 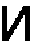 MeHTa 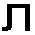 bHOMy ( 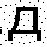 Oκ 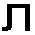 H 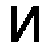 ecκoMy) 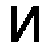 y 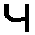 eH 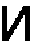 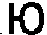 HOBbIX 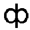 apMaκO 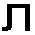 O 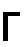 eCκ 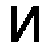 X Be 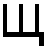 eCTB. 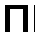 O 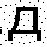 o 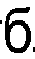 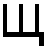 e 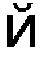 pe 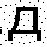 aκ 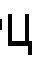 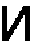 e 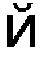 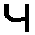 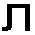 .-κOp. PAMH, 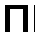 po 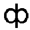 . P.Y. Xa 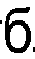 p 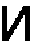 eBa.- 2 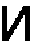 3 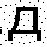 ., nepepa6. 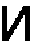 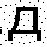 o 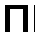 . - M, OAO 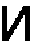 3 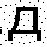 aTe 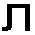 bCTBO «Me 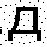 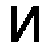 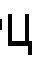 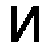 Ha» - 2005. (Guidelines for Experimental (Non-Clinical) Study of New Pharmaceutical Substances. Under the general editorship of Associate Member of RAMS professor R.U.Khabriyev. - 2nd edition, revised and enlarged - M., OAO Izdatelstvo Meditsyna - 2005)
19. 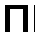 po 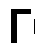 paMMa no yxo 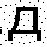 y 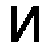 co 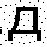 p 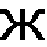 aH 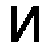 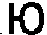 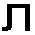 a6opaTOpHbIX 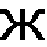 BOTHbIX H 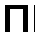 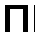 « 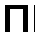 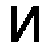 TOMH 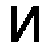 κ 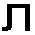 a 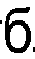 OpaTOpHbIX 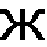 BOTHbIX» Φ 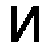 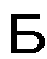 X PAH OT HO 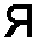 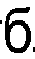 p 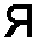 2005 r. (A Program for Care and Management of Laboratory Animals of the Nursery for laboratory animals of the Institute of Bioorganic Chemistry of the Russian Academy of Sciences, dated November, 2005)
20. PyκOBO 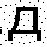 CTBO no 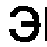 κC 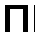 ep 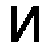 MeHTa 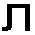 bHOMy ( 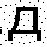 Oκ 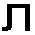 H 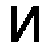 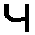 eCκOMy) 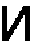 3y 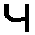 eH 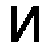 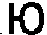 HOBbIX 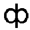 apMaκO 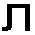 O 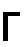 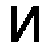 eCκ 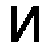 X Be 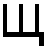 eCTB. 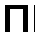 O 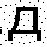 O 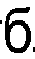 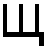 e 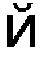 pe 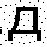 aκ 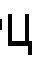 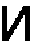 e 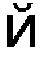 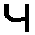 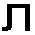 .-κOp. PAMH, 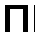 po 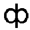 . P.Y. Xa 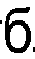 p 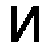 eBa.- 2 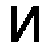 3 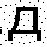 ., nepepa6. 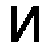 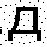 O 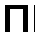 . - M, OAO 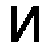 3 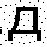 aTe 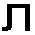 bCTBO «Me 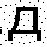 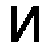 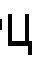 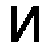 Ha» - 2005., c.100-122. (Guidelines for Experimental (Non-Clinical) Study of New Pharmaceutical Substances. Under the general editorship of Associate Member of RAMS professor R.U.Khabriyev. - 2nd edition, revised and enlarged - M., OAO Izdatelstvo Meditsyna - 2005, pages 100-122).

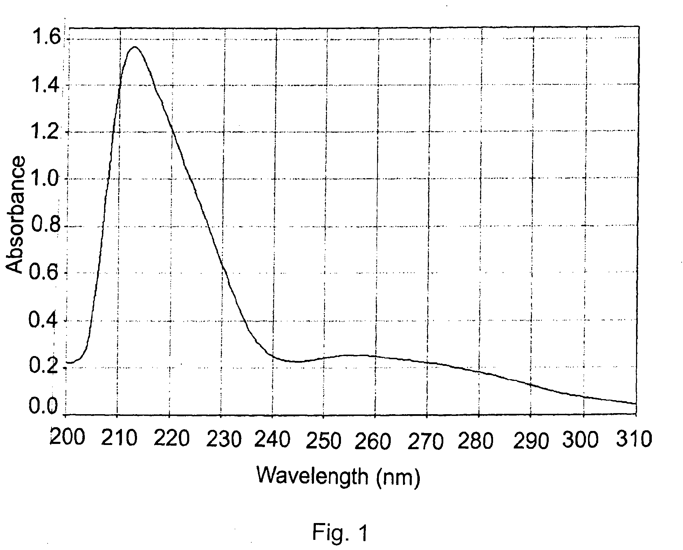

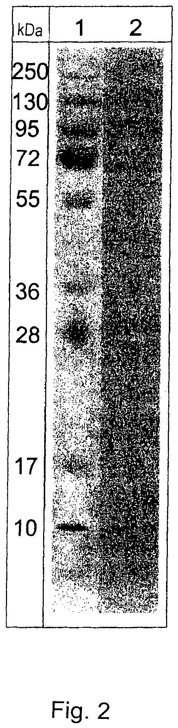

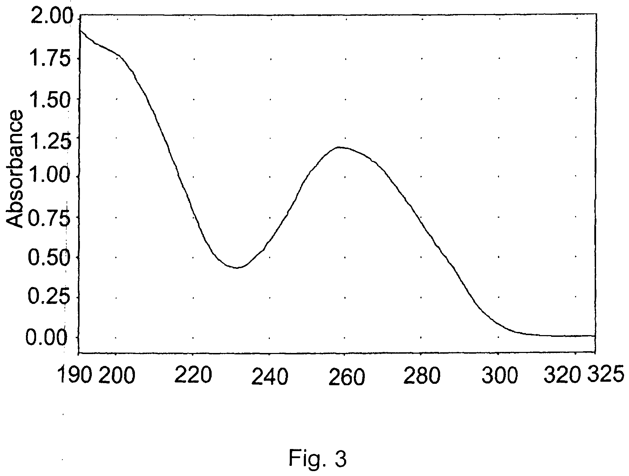

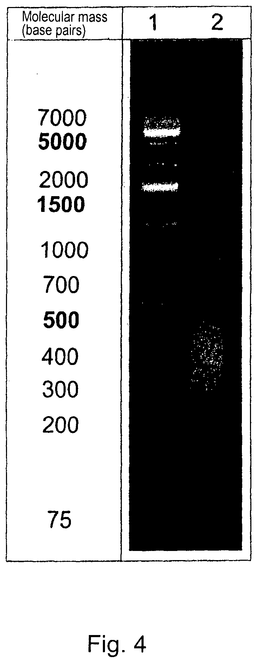

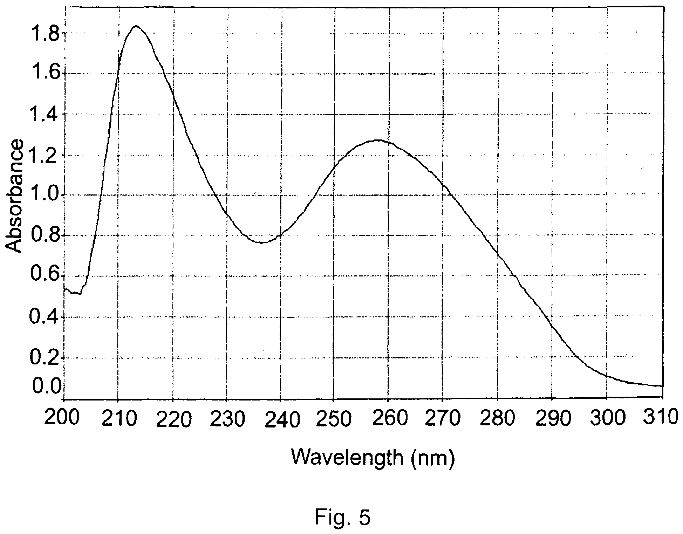

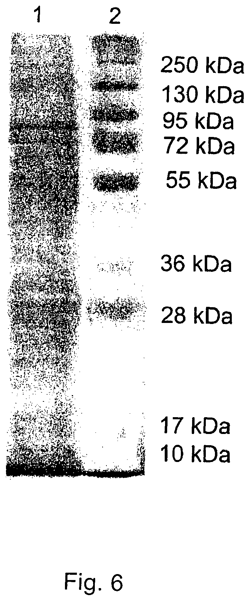

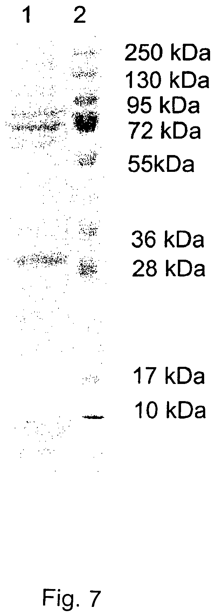

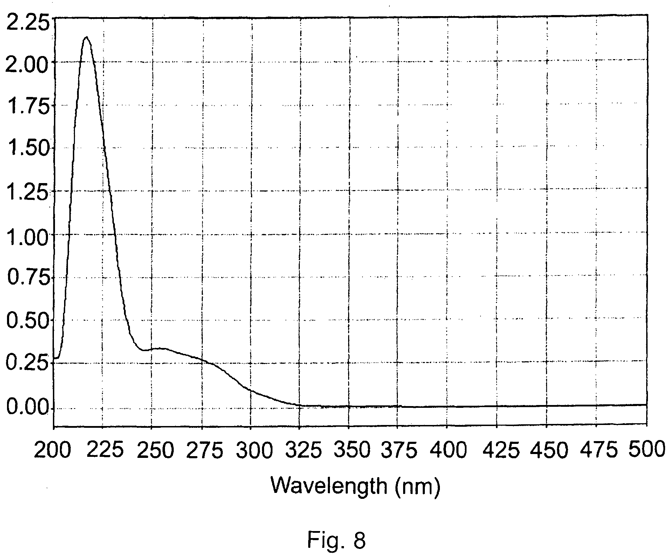

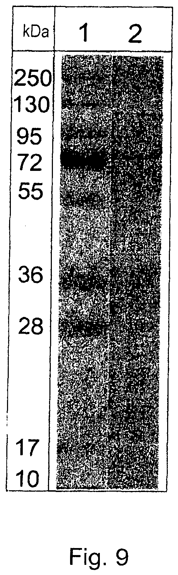

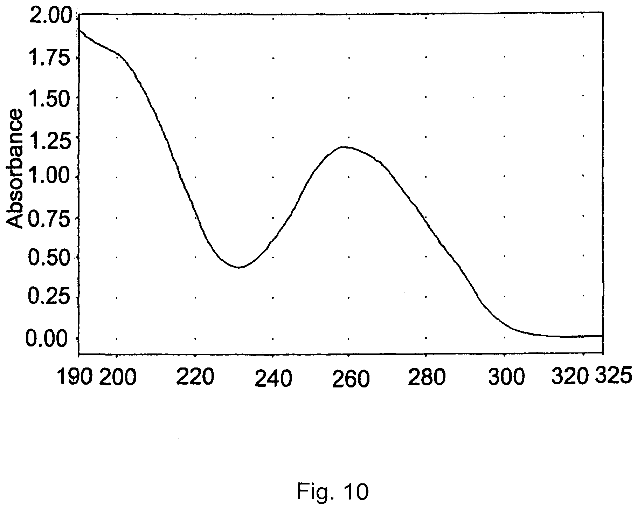

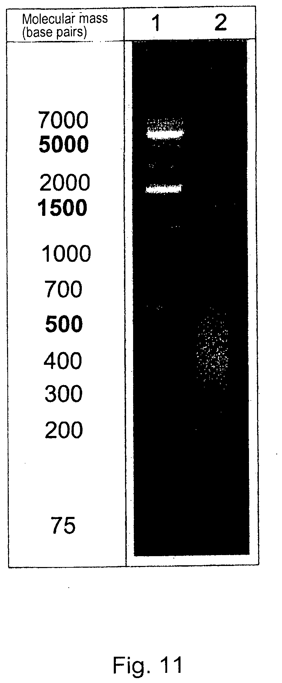

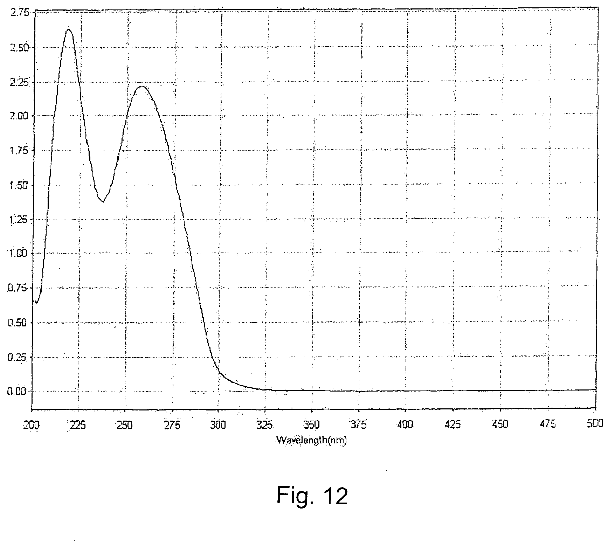

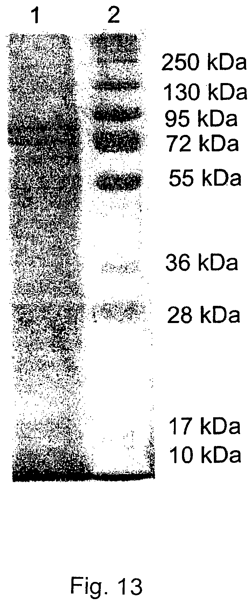

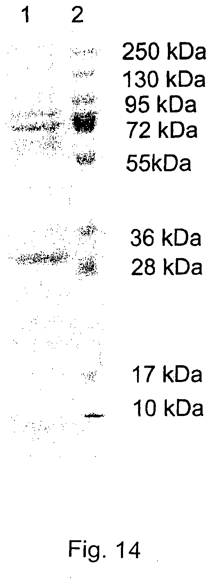

## Claims

1. A method for producing the protein/polypeptide complex, in which nerve and skin tissues of hoofed farm embryos of gestation stage from the middle of the first third to the middle of the last third of pregnancy are stepwise:
- homogenized in a buffer with a simultaneous extraction in the presence of reversible proteolysis inhibitors, nonionic detergents and solubilizers at the buffer : tissue ratio of at least 1:0.5, at minimum pH 5.2 and maximum pH 8.5, with further centrifugation at g ranging from 10 000 - 28 000 during 90 - 30 min;
- the resulting supernatant is filtered;
- the filtrate undergoes anion-exchange chromatography and separating associated proteins using an eluent with ionic strength ranging from 0.14 to 0.21 mmol/L at pH ranging from 5.2 to 8.5, starting to collect target fractions by a mobile phase at ionic strength of 0.175 mmol/L, increasing it smoothly or stepwise with 0.035 mmol/L increment up to 0.21 mmol/L ionic strength value, the resulting fractions are associated and undergo sterilization filtration.

2. A protein/polypeptide complex (PPC) produced using the method in accordance to the Claim 1, produced from nerve and skin tissues of hoofed farm embryos of gestation stage from the middle of the first third to the middle of the last third of pregnancy, including negatively charged faintly acid, neutral proteins and polypeptides classified as growth and differentiation factors, signaling molecules determining its biological and pharmacological activity, with molecular weights ranging from 0.5 to 200 kDa, therewith at least 70% of the total protein amount having molecular weights ranging from 20 to 180 kDa, having a specific set of bands at denaturing gel electrophoresis ("SDS-Page") in 5% polyacrylamide gel compared to the standard set of marker proteins having molecular weights ranging from 1 kDa to 250 kDa and an absorption peak at wavelength of 215±5 nm at ultraviolet spectrum recorded at wavelengths ranging from 200 to 500 nm.

3. A pharmaceutical composition for use in the treatment of autoimmune, vascular, toxic, infectious skin diseases, wherein the composition is prepared as a solution containing, as an active ingredient, the protein/polypeptide complex according to Claim 2 in concentration of 0.01 - 2.0 mg/ml, obtainable by the method according to Claim 1, polynucleotide fraction in the form of deoxyribonucleic acid sodium salt obtained from associated ballast tissue material and centrifugate after the centrifugation of filtered homogenate formed in the course of producing the protein/polypeptide complex using the method according to Claim 1 and having absorption peak at wavelength of 260 ± 2 nm and minimum absorption at wavelength of 260 ± 2 nm with ultraviolet spectrum recorded at wavelengths ranging from 190 to 325 nm.

4. A pharmaceutical composition for use according to Claim 3, **characterized in that** it is formulated for external, parenteral (intradermal, subcutaneous) and contact administration to skin and mucous membranes.

5. A pharmaceutical composition for use according to Claims 3-4, **characterized in that** it contains, as a carrier, a diluent in the form of a buffer solution and excipients including high-molecular compounds (polymers), stabilizers, detergents, preservatives and solubilizers.

6. A pharmaceutical composition for use according to Claim 3, **characterized in that** it contains, as polynucleotide components, polynucleotides in the form of medium- and low-molecule fractions of deoxyribonucleic acid sodium salt.

7. Nontherapeutic use of a composition for making tissue-specific, reparative and rejuvenating effect on skin tissue , wherein the composition is prepared as a solution containing, as an active ingredient, the protein/polypeptide complex according to Claim 2 in concentration of 0.01 - 2.0 mg/ml, obtainable by the method according to Claim 1, polynucleotide fraction in the form of deoxyribonucleic acid sodium salt obtained from associated ballast tissue material and centrifugate after the centrifugation of filtered homogenate formed in the course of producing the protein/polypeptide complex using the method according to Claim 1 and having absorption peak at wavelength of 260 ± 2 nm and minimum absorption at wavelength of 260 ± 2 nm with ultraviolet spectrum recorded at wavelengths ranging from 190 to 325 nm.

## Patentansprüche

1. Verfahren zur Herstellung des Protein/Polypeptid-Komplexes, in welchem Nerven- und Hautgewebe von Huftierzucht-Embryos des Trächtigkeitsstadiums von der Mitte des ersten Drittels bis zur Mitte des letzten Drittels der Schwangerschaft schrittweise
- in einem Puffer homogenisiert werden, mit einer gleichzeitigen Extraktion in Gegenwart reversibler Proteolyseinhibitoren, nichtionischer Detergenzien und Lösungsvermittler, bei dem Verhältnis Puffer : Gewebe von mindestens 1:0,5, bei einem minimalem pH 5,2 und maximalem pH 8,5, weiterhin mit Zentrifugation bei g im Bereich von 10 000 - 28 000 während 90-30 Minuten;
- der resultierende Überstand wird filtriert;
- das Filtrat wird einer Anionenaustauschchromatographie ausgesetzt und Auftrennen verbundener Proteine unter Verwendung eines Eluenten mit einer Ionenstärke im Bereich von 0,14 bis 0,21 mmol/L bei einem pH im Bereich von 5,2 bis 8,5, Beginnen, Zielfraktionen durch eine mobile Phase bei einer Ionenstärke von 0,175 mmol/L zu sammeln, wobei die Ionenstärke gleichmäßig oder schrittweise mit einem Inkrement von 0,035 mmol/L bis zu einem Wert für die Ionenstärke von 0,21 mmol/L gesteigert wird, die resultierenden Fraktionen werden vereint und einer Sterilfiltration ausgesetzt.

2. Protein/Polypeptid-Komplex (PPC), hergestellt unter Verwendung des Verfahrens nach Anspruch 1, hergestellt aus Nerven- und Hautgewebe von Huftierzucht-Embryos des Trächtigkeitsstadiums von der Mitte des ersten Drittels bis zur Mitte des letzten Drittels der Schwangerschaft, beinhaltend negativ geladene, leicht saure, neutrale Proteine und Polypeptide, klassifiziert als Wachstums- und Differenzierungsfaktoren, Signalmoleküle, die seine biologische und pharmakologische Aktivität bestimmen, mit Molgewichten im Bereich von 0,5 bis 200 kDa, damit mindestens 70% der gesamten Proteinmenge ein Molgewicht im Bereich von 20 - 180 kDa aufweist, mit einem spezifischen Satz an Banden in denaturierender Gelelektrophorese ("SDS-Page") in 5% Polyacrylamidgel, verglichen mit dem Standardsatz von Referenzproteinen mit Molgewichten im Bereich von 1 kDa bis 250 kDa, und einem Absorptionsmaximum bei Wellenlänge 215±5 nm in einem Ultraviolett-Spektrum, aufgenommen bei Wellenlängen im Bereich von 200 bis 500 nm.

3. Pharmazeutische Zusammensetzung zur Verwendung in der Behandlung von autoimmunen, vaskulären, toxischen, infektiösen Hautkrankheiten, wobei die Zusammensetzung als eine Lösung hergestellt wird, enthaltend als einen aktiven Inhaltsstoff in einer Konzentration von 0,01 - 2,0 mg/mL den Protein/Polypeptid-Komplex nach Anspruch 2, erhältlich durch das Verfahren nach Anspruch 1, eine Polynukleotidfraktion in Form eines Natriumsalzes von Desoxyribonukleinsäure, erhalten aus vereinigtem Ballastgewebematerial und Zentrifugat nach der Zentrifugation des filtrierten Homogenisats, welche im Laufe der Herstellung des Protein/Polypeptid-Komplexes unter Verwendung des Verfahrens nach Anspruch 1 gebildet werden, und ein Absorptionsmaximum bei Wellenlänge 260 ± 2 nm und minimale Absorption bei Wellenlänge 260 ± 2 nm aufweist, wobei ein ultraviolettes Spektrum bei Wellenlängen im Bereich von 190 bis 325 nm aufgenommen ist.

4. Pharmazeutische Zusammensetzung zur Verwendung nach Anspruch 3, **dadurch gekennzeichnet, dass** sie für externe, parenterale (intradermal, subkutan) und Kontakt-Verabreichung auf Haut und Schleimhäute formuliert ist.

5. Pharmazeutische Zusammensetzung zur Verwendung nach Ansprüchen 3-4, **dadurch gekennzeichnet, dass** sie als einen Träger, ein Verdünnungsmittel in Form einer Pufferlösung und Hilfsstoffe, inklusive hochmolekularer Verbindungen (Polymere), Stabilisatoren, Detergenzien, Konservierungsmittel und Lösungsvermittler, enthält.

6. Pharmazeutische Zusammensetzung zur Verwendung nach Anspruch 3, **dadurch gekennzeichnet, dass** sie als Polynukleotid-Bestandteile Polynukleotide in Form von mittel- und niedermolekularen Fraktionen aus Natriumsalz von Desoxyribonukleinsäure, enthält.

7. Nicht-therapeutische Verwendung einer Zusammensetzung, um einen gewebsspezifischen, reparativen und verjüngenden Effekt auf Hautgewebe zu erreichen, wobei die Zusammensetzung als eine Lösung hergestellt wird, enthaltend als einen aktiven Inhaltsstoff in einer Konzentration von 0,01 - 2,0 mg/mL den Protein/Polypeptid-Komplex nach Anspruch 2, erhältlich durch das Verfahren nach Anspruch 1, eine Polynukleotidfraktion in Form von Natriumsalz von Desoxyribonukleinsäure, erhalten aus vereinigtem Ballastgewebematerial und Zentrifugat nach der Zentrifugation des filtrierten Homogenisats, welche im Laufe der Herstellung des Protein/Polypeptid-Komplexes unter Verwendung des Verfahrens nach Anspruch 1 gebildet werden, und ein Absorptionsmaximum bei Wellenlänge 260 ± 2 nm und minimale Absorption bei Wellenlänge 260 ± 2 nm aufweist, wobei das ultraviolette Spektrum bei Wellenlängen im Bereich von 190 bis 325 nm aufgenommen ist.

## Revendications

1. Procédé de production du complexe protéine/ polypeptide, dans lequel des tissus nerveux et de peau d'embryons de ferme d'ongulés du stade de gestation du milieu du premier tiers au milieu du dernier tiers de grossesse sont par étape :
- homogénéisés dans un tampon avec une extraction simultanée en présence d'inhibiteurs de protéolyse réversibles, de détergents non ioniques et de solubilisants au rapport tampon:tissu d'au moins 1:0,5, à un pH minimum de 5,2 et un pH maximum de 8,5, avec en outre une centrifugation à g se situant dans la plage allant de 10 000 à 28000 durant 90 à 30 minutes ;
- le surnageant obtenu est filtré ;
- le filtrat subit une chromatographie d'échange d'anions et la séparation des protéines associées en utilisant un éluant avec une force ionique se situant dans la plage allant de 0,14 à 0,21 mmol/l à un pH se situant dans la plage allant de 5,2 à 8,5, en commençant à collecter les fractions cibles par une phase mobile à une force ionique de 0,175 mmol/l, en l'augmentant doucement ou par étape avec un incrément de 0,035 mmol/l jusqu'à 0,21 mmol/l de valeur de force ionique, les fractions obtenues sont associées et subissent une filtration de stérilisation.

2. Complexe protéine/polypeptide (PPC) produit en utilisant le procédé selon la revendication 1, produit à partir de tissus nerveux et de peau d'embryons de ferme d'ongulés de stade de gestation du milieu de premier tiers au milieu de dernier tiers de grossesse, comprenant un acide légèrement chargé négativement, des protéines neutres et des polypeptides classés comme facteurs de croissance et de différenciation, des molécules de signalisation déterminant son activité biologique et pharmacologique, avec des poids moléculaires se situant dans la plage allant de 0,5 à 200 kDa, avec au moins 70 % de la quantité de protéines totale ayant des poids moléculaires se situant dans la plage allant de 20 à 180 kDa, ayant un jeu de bandes spécifiques en électrophorèse sur gel dénaturant (« SDS-Page ») dans un gel à 5 % de polyacrylamide par rapport au jeu standard de protéines marqueurs ayant des poids moléculaires se situant dans la plage allant de 1 à 250 kDa et un pic d'absorption à une longueur d'onde de 260 ± 5 nm à un spectre d'ultraviolet enregistré à des longueurs d'onde se situant dans la plage allant de 200 à 500 nm.

3. Composition pharmaceutique destinée à être utilisée dans le traitement de maladies de la peau infectieuses, toxiques, vasculaires, autoimmunes, la composition étant préparée comme une solution contenant, comme ingrédient actif, le complexe protéine/polypeptide selon la revendication 2 à une concentration de 0,01 à 2,0 mg/ml que l'on peut obtenir par le procédé selon la revendication 1, une fraction de polynucléotide sous la forme de sel de sodium d'acide désoxyribonucléique obtenu d'un matériau de tissu de lest associé et du centrifugeat obtenu après la centrifugation de l'homogénat filtré formé au cours de la production du complexe protéine/polypeptide en utilisant le procédé selon la revendication 1 et ayant un pic d'absorption à une longueur d'onde de 260 ± 2 nm et un pic d'absorption minimum à une longueur d'onde de 260 ± 2 nm avec un spectre d'ultraviolet enregistré à des longueurs d'onde se situant dans la plage allant de 190 à 325 nm.

4. Composition pharmaceutique destinée à être utilisée selon la revendication 3, **caractérisée en ce qu'**elle est formulée pour l'administration externe, parentérale (intradermique, sous-cutanée) et de contact à la peau et aux membranes muqueuses.

5. Composition pharmaceutique destinée à être utilisée selon les revendications 3 et 4, **caractérisé en ce qu'**elle contient, comme support, un diluant sous la forme d'une solution tampon et des excipients comprenant des composés de haut poids moléculaire (polymères), des stabilisants, des détergents, des conservateurs et des solubilisants.

6. Composition pharmaceutique destinée à être utilisée selon la revendication 3, **caractérisée en ce qu'**elle contient, comme composants polynucléotidiques, des poly-nucléotides sous forme de fractions moléculaires de milieu et faible de sel de sodium d'acide désoxyribonuléique.

7. Usage non thérapeutique d'une composition pour rendre un effet spécifique de tissu, réparateur et rajeunissant sur du tissu de peau, la composition étant préparée comme une solution contenant, comme ingrédient actif, le complexe protéine/polypeptide selon la revendication 2 à une concentration de 0,01 à 2,0 mg/ml, que l'on peut obtenir par le procédé selon la revendication 1, une fraction polynucléotidique sous la forme de sel de sodium d'acide désoxyribonucléique obtenu à partir d'un matériau de tissu de lest associé et du centrifugeat après la centrifugation de l'homogénat filtré formé au cours de la production du complexe protéine/polypeptide en utilisant le procédé selon la revendication 1 et ayant un pic d'absorption à une longueur d'onde de 260 ± 2 nm et une absorption minimum à une longueur d'onde de 260 ± 2 nm avec un spectre d'ultraviolet enregistré à des longueurs d'onde se situant dans la plage allant de 190 à 325 nm.
